(19) **Europäisches Patentamt**
**European Patent Office**
**Office européen des brevets**

(11) **EP 4 289 338 A1**

(12) **EUROPEAN PATENT APPLICATION**
published in accordance with Art. 153(4) EPC

(43) Date of publication:
**13.12.2023 Bulletin 2023/50**

(21) Application number: **21924654.3**

(22) Date of filing: **05.02.2021**

(51) International Patent Classification (IPC):
***A61B 3/10*** *(2006.01)*

(52) Cooperative Patent Classification (CPC):
**A61B 3/10**

(86) International application number:
**PCT/JP2021/004244**

(87) International publication number:
**WO 2022/168259 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TOPCON CORPORATION**
**Tokyo 174-8580 (JP)**

(72) Inventors:
• **MINO Toshihiro**
**Tokyo 174-8580 (JP)**

• **YEH Shuyun**
**Tokyo 174-8580 (JP)**
• **NAKAJIMA Masashi**
**Tokyo 174-8580 (JP)**
• **FUKUMA Yasufumi**
**Tokyo 174-8580 (JP)**

(74) Representative: **Gagel, Roland**
**Patentanwalt Dr. Roland Gagel**
**Landsberger Strasse 480a**
**81241 München (DE)**

(54) **OPHTHALMIC INFORMATION PROCESSING DEVICE, OPHTHALMIC DEVICE, OPHTHALMIC INFORMATION PROCESSING METHOD, AND PROGRAM**

(57)     This ophthalmic information processing device includes an acquisition unit and a normalization unit. The acquisition unit acquires eye shape data or an intraocular distance of an eye of a subject. The normalization unit normalizes the eye shape data or the intraocular distance on the basis of body data of the subject or the degree of refraction of the eye of the subject.

FIG. 5

**EP 4 289 338 A1**

**Description**

[TECHNICAL FIELD]

The present invention relates to an ophthalmic information processing apparatus, an ophthalmic apparatus, an ophthalmic information processing method, and a program.

[BACKGROUND ART]

Suppression of myopia progression is necessary to maintain QOV (Quality Of Vision) and QOL (Quality Of Life) in the future. In particular, high myopia and pathological myopia have become one of the major causes of blindness, and various treatment methods to suppress myopia progression have been paid attention.

It is believed that myopia progression is related to how the eye shape changes (Non-Patent Document 1, Non-Patent Document 2). By grasping the eye shape or a change in the eye shape, an appropriate treatment method corresponding to the eye shape or the change in the eye shape may be selected. For example, Non-Patent Document 1 discloses various types of the eye shape. By identifying the type of the eye shape, an appropriate treatment method corresponding to the identified type may be selected.

[PRIOR ART DOCUMENTS]

[NON-PATENT DOCUMENTS]

[NON-PATENT DOCUMENT 1] Verkicharla PK et al., "Eye shape and retinal shape, and their relation to peripheral refraction", Ophthalmic Physiol Opt 2012, 32, 184-199. doi: 10.1111/j.1475-1313.2012.00906.x

[NON-PATENT DOCUMENT 2] Muka Moriyama et al., "Quantitative Analyses of High-Resolution 3D MR Images of Highly Myopic Eyes to Determine Their Shapes", Investigative Ophthalmology & Visual Science, July 2012, Vol. 53, No. 8, pp.4510-4518

[SUMMARY OF THE INVENTION]

[PROBLEMS TO BE SOLVED BY THE INVENTION]

The eye shape changes with growth. Therefore, it is very difficult to determine whether the change(s) in eye shape is due to growth or myopia progression, and the eye shape or the change(s) in the eye shape cannot be determined reproducibly and with high precision.

The present invention has been made in view of such circumstances, and one of objects of the present invention is to provide a new technique for identifying an eye shape or a change in the eye shape reproducibly and with high precision.

[MEANS OF SOLVING THE PROBLEMS]

One aspect of some embodiments is an ophthalmic information processing apparatus, including: an acquisition unit configured to acquire eye shape data or an intraocular distance of an eye of a subject; and a normalizer configured to normalize the eye shape data or the intraocular distance based on body data of the subject or a refractive power of the eye of the subj ect.

Another aspect of some embodiments is an ophthalmic information processing apparatus, including: a normalizer configured to normalize measurement data of an eye of a subject based on body data of the subject or a refractive power of the eye of the subject; and a calculator configured to calculate normalized eye shape data or a normalized intraocular distance of the eye of the subject based on the measurement data normalized by the normalizer.

Still another aspect of some embodiments is an ophthalmic apparatus, including: a measurement system configured to measure an eye shape or an intraocular distance of the eye of the subject; and the ophthalmic information processing apparatus of any one of the above.

Still another aspect of some embodiments is an ophthalmic information processing method, including: an acquisition step of acquiring eye shape data or an intraocular distance of an eye of a subject; and a normalization step of normalizing the eye shape data or the intraocular distance based on body data of the subject or a refractive power of the eye of the subject.

Still another aspect of some embodiments is an ophthalmic information processing method, including: a normalization step of normalizing measurement data of an eye of a subject based on body data of the subject or a refractive power of the eye of the subject; and a calculation step of calculating normalized eye shape data or a normalized intraocular distance of the eye of the subject based on the measurement data normalized in the normalization step.

Still another aspect of some embodiments is a program of causing a computer to execute each step of the ophthalmic information processing method of any one of the above.

[EFFECTS OF THE INVENTION]

According to the present invention, a new technique for identifying an eye shape or a change in the eye shape reproducibly and with high precision can be provided.

[BRIEF EXPLANATION OF THE DRAWINGS]

[FIG. 1] FIG. 1 is a schematic diagram illustrating an example of a configuration of an optical system of an ophthalmic apparatus according to embodiments.

[FIG. 2] FIG. 2 is a schematic diagram illustrating an example of a configuration of an optical system of the ophthalmic apparatus according to the embodiments.

[FIG. 3] FIG. 3 is a schematic diagram illustrating an example of a configuration of a processing system of the ophthalmic apparatus according to the embodiments.

[FIG. 4] FIG. 4 is a schematic diagram illustrating an example of a configuration of a processing system of the ophthalmic apparatus according to the embodiments.

[FIG. 5] FIG. 5 is a schematic diagram illustrating an example of a configuration of a processing system of the ophthalmic apparatus according to the embodiments.

[FIG. 6] FIG. 6 is a schematic diagram illustrating an example of a configuration of a processing system of the ophthalmic apparatus according to the embodiments.

[FIG. 7] FIG. 7 is a schematic diagram for explaining an operation of the ophthalmic apparatus according to the embodiments.

[FIG. 8] FIG. 8 is a schematic diagram for explaining an operation of the ophthalmic apparatus according to the embodiments.

[FIG. 9] FIG. 9 is a schematic diagram for explaining an operation of the ophthalmic apparatus according to the embodiments.

[FIG. 10] FIG. 10 is a schematic diagram for explaining an operation of the ophthalmic apparatus according to the embodiments.

[FIG. 11A] FIG. 11A is a schematic diagram for explaining an operation of the ophthalmic apparatus according to the embodiments.

[FIG. 11B] FIG. 11B is a schematic diagram for explaining an operation of the ophthalmic apparatus according to the embodiments.

[FIG. 11C] FIG. 11C is a schematic diagram for explaining the operation of the ophthalmic apparatus according to the embodiments.

[FIG. 12] FIG. 12 is a schematic diagram illustrating a flow of an example of an operation of the ophthalmic apparatus according to the embodiments.

[FIG. 13] FIG. 13 is a schematic diagram illustrating a flow of an example of an operation of the ophthalmic apparatus according to the embodiments.

[FIG. 14] FIG. 14 is a schematic diagram illustrating a flow of an example of an operation of the ophthalmic apparatus according to the embodiments.

[FIG. 15] FIG. 15 is a schematic diagram illustrating a flow of an example of an operation of the ophthalmic apparatus according to the embodiments.

[FIG. 16] FIG. 16 is a schematic diagram illustrating a flow of an example of an operation of the ophthalmic apparatus according to the embodiments.

[FIG. 17] FIG. 17 is a schematic diagram illustrating a flow of an example of an operation of the ophthalmic apparatus according to the embodiments.

[FIG. 18A] FIG. 18A is a schematic diagram for explaining an operation of the ophthalmic apparatus according to the embodiments.

[FIG. 18B] FIG. 18B is a schematic diagram for explaining an operation of the ophthalmic apparatus according to the embodiments.

[MODES FOR CARRYING OUT THE INVENTION]

Referring now to the drawings, exemplary embodiments of an ophthalmic information processing apparatus, an ophthalmic apparatus, an ophthalmic information processing method, and a program according to the present invention are described below. Any of the contents of the documents cited in the present specification and arbitrary known techniques may be applied to the embodiments below.

It is believed that myopia progression is related to how the eye shape changes. However, it is known that the eye shape is not strictly true spherical and may change due to the growth of the subject (the eye of the subject) or lead to myopia progression due to factors other than growth.

The ophthalmic information processing apparatus according to embodiments is configured to normalize data representing eye (ocular) shape (or intraocular distance) calculated from measurement data obtained by measuring the subject's eye, using parameter(s) changing due to the growth of the subject or a refractive power (dioptric power) of the subject's eye, to acquire normalized data. Alternatively, the ophthalmic information processing apparatus according to the embodiments is configured to normalize the measurement data by performing scaling processing on the measurement data using the parameter(s) changing due to the growth of the subject or the refractive power of the subject's eye, and to calculate normalized data representing the eye shape (or intraocular distance) from the normalized measurement data.

The normalized data acquired by normalizing using the parameter(s) changing due to the growth of the subject or the refractive power of the subject's eye in this way represents the degree of change in the eye shape (or intraocular distance) due to factors other than the growth. By using the normalized data or time-series data of the normalize data as an indicator, the eye shape or the change in the eye shape can be identified reproducibly and with high precision, without being affected by growth factors.

In some embodiments, the eye shape type (change pattern) is classified based on the normalized data, and a treatment policy corresponding to the classified eye shape type is determined. In some embodiments, a future change in the eye shape is estimated based on the normalized data.

Examples of the data representing the eye shape include data representing a shape of an anterior segment, data representing a shape of a posterior segment, data representing a shape of an equatorial part (part between the anterior segment and the posterior segment) of the eye, and data representing an outer wall of the eye. Examples of the data representing the shape of the anterior segment include data (corneal curvature, corneal radius of curvature) representing a shape of a cornea. Examples of the data representing the shape of the posterior segment include data (fundus curvature, fundus radius of curvature) representing a shape of a predetermined layer region in the fundus (retina). Examples of the data representing the shape of the equatorial part of the eye include data (scleral curvature, scleral radius of curvature) representing a shape of a sclera at the equatorial part. Examples of the data representing the shape of the outer wall of the eye include data representing the shape of the sclera.

Examples of the intraocular distance include a distance between predetermined sites in the anterior segment (e.g., distance from a corneal apex to a predetermined position on the anterior surface of cornea), a distance between predetermined sites in the posterior segment (e.g., distance between a macula and an optic disc), a distance between predetermined layer regions in the fundus, and an axial length.

The measurement data is acquired using an ophthalmic apparatus. The measurement data is acquired using a known corneal shape measurement apparatus (keratometer, placido corneal shape measurement apparatus), an anterior segment image analysis apparatus, an anterior segment optical coherence tomography (OCT) apparatus, or the like. The measurement data of the equatorial part and the measurement data of the posterior segment are acquired using an OCT apparatus.

Examples of the parameter(s) changing due to the growth of the subject include body data. Examples of the body data include a body length of the subject, a body weight of the subject, a head size (circumference length, volume), an eye socket size (longitudinal length of the socket, lateral length of the socket, depth of the socket), an axial length, a corneal curvature (corneal radius of curvature), a pupillary distance, and a corneal diameter (White To White: WTW). In case of normalizing the data representing the shape of the anterior segment, parameter(s) other than corneal curvature is/are employed as parameter(s) changing due to the growth of the subject. It should be noted that when the intraocular distance (e.g., axial length) changes due to the growth of the subject, the body data may include such intraocular distance. Hereafter, the data of the subject that changes due to the growth of the subject, including those included in the intraocular distance, may be referred to as "body data".

In the following, the shape of the anterior segment is simplified by assuming that the original eye is spherical (true sphere), and a where the ophthalmic information processing apparatus according to the embodiments normalizes the fundus radius of curvature or the fundus curvature, which represents the shape of the fundus of the subject's eye, using the axial length to acquire normalized data representing the degree of change in the shape of the eye will be described. A shape of the retinal pigment epithelium (RPE) layer in the retina is used as an example of the shape of the fundus.

The ophthalmic apparatus according to the embodiments realizes the function(s) of the ophthalmic information processing apparatus according to the embodiments. An ophthalmic information processing method according to the embodiments is realized by the ophthalmic information processing apparatus according to the embodiments. A program according to the embodiments causes a processor (computer) to execute each step of the ophthalmic information processing method according to the embodiments. A recording medium according to the embodiments is a computer readable non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the functions according to the embodiments by reading out computer program(s) stored in a storage circuit or a storage device and executing the computer program(s).

Hereinafter, the ophthalmic apparatus that realizes the functions of the ophthalmic information processing apparatus according to the embodiments will be described. A case where the ophthalmic apparatus according to the embodiments includes an OCT optical system configured to perform OCT on the subject's eye will be described. However, the configuration of the ophthalmic apparatus according to the embodiments is not limited thereto. For example, the ophthalmic apparatus according to the embodiments may be configured to acquire the measurement data of the subject's eye from an external OCT apparatus.

The ophthalmic apparatus according to some embodiments has not only a function of an OCT apparatus for performing OCT, but also functions of at least one of a fundus camera, a scanning laser ophthalmoscope, a slit lamp microscope, and a surgical microscope. Further, the ophthalmic apparatus has a function of measuring optical characteristics of the subject's eye. Examples of the ophthalmic apparatus having the function of measuring optical characteristics of the subject's eye include a tonometer, a wave front analyzer, a specular microscope, a perimeter, and the like. The ophthalmic apparatus according to some embodiments has a function of a laser treatment apparatus used for laser treatment.

Hereinafter, in the embodiments, a case of using the spectral domain type OCT method in the measurement and the like using OCT will be described particularly in detail. However, the configuration according to the embodiments can also be applied to an ophthalmic apparatus using other type of OCT (for example, swept source type OCT).

< Configuration of optical system >

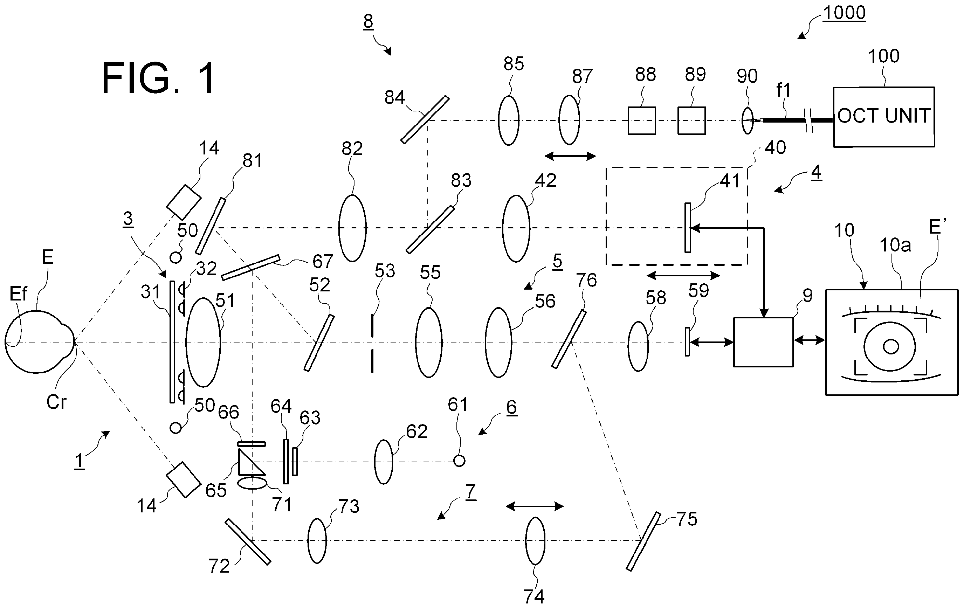

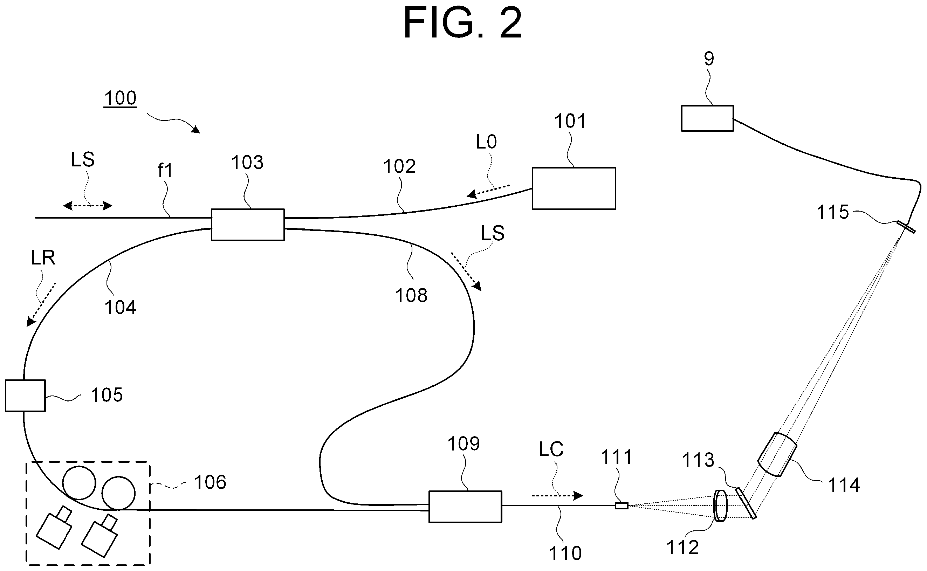

FIG. 1 and FIG. 2 show examples of a configuration of an optical system of an ophthalmic apparatus according to embodiments. FIG. 1 represents an example of the entire configuration of the optical system of the ophthalmic apparatus 1000 according to the embodiments. FIG. 2 represents an example of the configuration of an OCT unit 100 in FIG. 1.

The ophthalmic apparatus 1000 according to the embodiments includes an optical system for observing the subject's eye E, an optical system for inspecting the subject's eye E, and a dichroic mirror that wavelength-separates the optical paths of these optical systems. An anterior segment observation system 5 is provided as the optical system for observing the subject's eye E. An OCT optical system, a refractometry optical system (refractive power measurement optical system), and the like are provided as the optical system for inspecting the subject's eye E.

The ophthalmic apparatus 1000 includes an alignment system 1, a keratometry system 3, a fixation projection system 4, an anterior segment observation system 5, a refractometry projection system 6, a refractometry light reception system 7, and an OCT optical system 8. Hereinafter, for example, it is assumed that light with 940 nm to 1000 nm is used in the anterior segment observation system 5, light with 850 nm to 880 nm is used in the refractometry optical system (refractometry projection system 6, refractometry light reception system 7), and light with 400 nm to 700 nm is used in fixation projection system 4. It is also assumed that light with 840 nm is used in the OCT optical system 8. In other words, it is assumed that the OCT optical system 8 performs OCT measurement (OCT scan) with 840 nm as the measurement wavelength.

( Alignment system 1 )

The alignment system 1 is used for Z alignment and XY alignment of the optical system relative to the subject's eye E. The Z alignment is an alignment in Z direction (front-back direction, working distance direction) parallel to an optical axis of the anterior segment observation system 5 (for example, objective lens 51). The XY alignment is an alignment in a direction (left-right direction (X direction), up-down direction (Y direction)) perpendicular to this optical axis.

The alignment system 1 includes two or more anterior segment cameras 14. The two or more anterior segment cameras 14 are configured to photograph the anterior segment of the subject's eye E from different directions substantially at the same time, and to output acquired two or more photographic images (anterior segment images) to a processor 9 described below. The processor 9 is configured to analyze the two or more photographic images from the two or more anterior segment cameras 14 to identify a three-dimensional position of the subject's eye E (specifically, characteristic site of the subject's eye E), and to perform alignment of the optical system relative to the subject's eye E (characteristic site) in the XY direction and the Z direction, based on the identified three-dimensional position.

Hereinafter, it is assumed that the alignment system 1 includes two anterior segment cameras 14. However, the alignment system 1 may include three or more anterior segment cameras 14. Alternatively, the function of one of the two anterior segment cameras 14 may be realized using an imaging element 59 provided in the anterior segment observation system 5 described below.

( Anterior segment observation system 5 )

The anterior segment observation system 5 is configured to acquire a moving image of an anterior segment of the subject's eye E. In an optical system through the anterior segment observation system 5, an imaging plane of the imaging element 59 is arranged at a pupil conjugate position (position substantially conjugate optically to a pupil of the subject's eye E). An anterior segment illumination light source 50 irradiates illumination light (for example, infrared light) onto the anterior segment of the subject's eye E. Light reflected from the anterior segment of the subject's eye E passes through an objective lens 51, is transmitted through a dichroic mirror 52, passes through an hole part formed in a diaphragm (telecentric diaphragm) 53, passes through relay lenses 55 and 56, and is transmitted through a dichroic mirror 76. The dichroic mirror 52 couples (separates) an optical path of the refractometry optical system and an optical path of the anterior segment observation system 5. The dichroic mirror 52 is disposed so that an optical path coupling plane for coupling these optical paths is inclined with respect to the optical axis of the objective lens 51. The light transmitted through the dichroic mirror 76 forms an image on the imaging plane of the imaging element 59 (area sensor) via an imaging lens 58. The imaging element 59 performs photographing and a signal outputting at a predetermined rate. The output (video signal) of the imaging element 59 is input to the processor 9 described below. The processor 9 displays an anterior segment image E' based on this video signal on a display screen 10a of a display unit 10 described below. The anterior segment image E' is an infrared moving image for example.

( Keratometry system 3 )

The keratometry system 3 is configured to project a ring-shaped light flux (infrared light) for measuring a shape of a cornea Cr of the subject's eye E onto the cornea Cr. A keratometry plate (kerato plate) 31 is disposed between the objective lens 51 and the subject's eye E. A keratometry ring light source 32 is provided on the back side (the objective lens 51 side) of the keratometry plate 31. In the keratometry plate 31, a keratometry (kerato) pattern (transmitting part) that transmits light from the keratometry ring light source 32 is formed along a circumference around the optical axis of the objective lens 51. It should be noted that the keratometry pattern may be formed in an arc shape (a part of the circumference) around the optical axis of the obj ective lens 51. By illuminating the keratometry plate 31 with light from the keratometry ring light source 32, the ring-shaped light flux (arc-like or circumferential (circular) measurement pattern) is projected onto the cornea Cr. The reflected light (keratometry ring image) from the cornea Cr of the subject's eye E is detected by the imaging element 59 along with the anterior segment image E'. The processor 9 calculates a corneal shape parameter representing a shape of the cornea Cr, by performing a known calculation based on this keratometry ring image.

( Refractometry projection system 6 and Refractometry light reception system 7 )

The refractometry optical system includes the refractometry projection system 6 and the refractometry light reception system 7 which are used for refractive power measurement. The refractometry projection system 6 is configured to project light flux (a ring-shaped light flux, for example) (infrared light) for measuring refractive power onto the fundus Ef. The refractometry light reception system 7 is configured to receive returning light of the light flux from the subject's eye E. The refractometry projection system 6 is provided in an optical path branched by a perforated prism 65 provided in an optical path of the refractometry light reception system 7. A hole part formed in the perforated prism 65 is arranged at the pupil conjugate position. In an optical system through the refractometry light reception system 7, the imaging plane of the imaging element 59 is arranged at a fundus conjugate position (position substantially conjugate optically to the fundus Ef of the subject's eye E).

In some embodiments, the refractometry light source 61 is a SLD (Super Luminescent Diode) light source which is a high-intensity light source. The refractometry light source 61 is movable in an optical axis direction. The refractometry light source 61 is arranged at the fundus conjugate position. Light emitted from the refractometry light source 61 passes through the relay lens 62 and is incident on a conical surface of the conical prism 63. The light incident on the conical surface is deflected and emits from a bottom surface of the conical prism 63. The light emitted from the bottom surface of the conical prism 63 passes through a ring-shaped light transmission part formed in a ring diaphragm 64. The light (ring-shaped light flux) passing through the light transmission part of the ring diaphragm 64 is reflected on a reflective surface formed around the hole part of the perforated prism 65, passes through a rotary prism 66, and is reflected by the dichroic mirror 67. The light reflected by the dichroic mirror 67 is reflected by the dichroic mirror 52, passes through the objective lens 51, and is projected onto the subject's eye E. The rotary prism 66 is used for averaging the light quantity distribution of the ring-shaped light flux with respect to the blood vessel or the diseased site of the fundus Ef or for reducing the speckle noise caused by the light source.

Returning light of the ring-shaped light flux projected onto the fundus Ef passes through the objective lens 51, and is reflected by the dichroic mirrors 52 and 67. The returning light reflected by the dichroic mirror 67 passes through the rotary prism 66, passes through the hole part of the perforated prism 65, passes through a relay lens 71, is reflected by a reflective mirror 72, and passes through a relay lens 73 and a focusing lens 74. The focusing lens 74 is movable along an optical axis of the refractometry light reception system 7. The light passing through the focusing lens 74 is reflected by the reflective mirror 75, is reflected by a dichroic mirror 76, and forms an image on the imaging plane of the imaging element 59 via the imaging lens 58. The processor 9 calculates a refractive power value of the subject's eye E by performing the known calculation based on the output of the imaging element 59. For example, the refractive power value includes a spherical power, an astigmatic power, and an astigmatic axis angle, or an equivalent spherical power.

( Fixation projection system 4 )

The OCT optical system 8, which will be described below, is provided in the optical path wavelength-separated from the optical path of the refractometry optical system by the dichroic mirror 67. The fixation projection system 4 is provided in the optical path branched from the optical path of the OCT optical system 8 by the dichroic mirror 83.

The fixation projection system 4 is configured to present a fixation target to the subject's eye E. A fixation unit 40 is disposed in the optical path of the fixation projection system 4. The fixation unit 40 is movable along an optical axis of the fixation projection system 4 under the control of the processing unit 9 described below. The fixation unit 40 includes a liquid crystal panel 41. A relay lens 42 is arranged between the dichroic mirror 83 ant the fixation unit 40.

Under the control of the processor 9, the liquid crystal panel 41 displays a pattern representing the fixation target. By changing the display position of the fixation target on the screen of the liquid crystal panel 41, the fixation position of the subject's eye E can be changed. Examples of the fixation position of the subject's eye E include a position for acquiring an image centered at a macular region of the fundus Ef, a position for acquiring an image centered at an optic disc, and a position for acquiring an image centered at the fundus center between the macular region and the optic disc. The display position of the pattern representing the fixation target can be arbitrarily changed.

Light from the liquid crystal panel 41 passes through the relay lens 42, is transmitted through the dichroic mirror 83, passes through a relay lens 82, is reflected by a reflective mirror 81, is transmitted through the dichroic mirror 67, and is reflected by the dichroic mirror 52. The light reflected by the dichroic mirror 52 passes through the objective lens 51 and is projected onto a fundus Ef. In some embodiment, each of the liquid crystal panel 41 and the relay lens 42 is independently movable in the optical axis direction.

( OCT optical system 8 )

The OCT optical system 8 is an optical system for performing OCT measurement. The position of the focusing lens 87 is adjusted so that an end face of an optical fiber f1 is conjugate to the optical system and the OCT measurement site based on the result of the refractometry performed before the OCT measurement. The OCT measurement site may be any part of the subject's eye E, such as the fundus Ef, the anterior segment.

The OCT optical system 8 is provided on the optical path wavelength-separated from the optical path of the refractometry optical system by the dichroic mirror 67. The optical path of the above fixation projection system 4 is coupled with the optical path of the OCT optical system 8 by the dichroic mirror 83. Thereby, the optical axes of the OCT optical system 8 and the fixation projection system 4 can be coupled coaxially.

The OCT optical system 8 includes an OCT unit 100. As shown in FIG. 2, the OCT unit 100 is provided with an optical system for performing OCT measurement (OCT imaging, OCT scan) on the subject's eye E. This optical system has a configuration similar to that of a conventional spectral domain type OCT apparatus. In other words, this optical system is configured to: split light (low coherence light) from a broadband light source into reference light and measurement light; make the measurement light having traveled through the subject's eye E (OCT measurement site) and the reference light having traveled through a reference optical path interfere with each other to generate interference light; and detect spectral components of the interference light. The result of the detection (detection signal) is sent to the processor 9.

A light source unit 101 emits broadband, low coherence light L0. The low coherence light L0, for example, has a wavelength components of a wavelength band (e.g., about 800 nm to 900 nm) in the near-infrared region, and has a temporal coherence length of about several tens of micrometers. In addition, near-infrared light with a wavelength range that is not visible to the human eye, for example, 1040 nm to 1060 nm may be used as the low coherence light L0.

Hereafter, the light source unit 101 is assumed to output the low coherence light L0 with a wavelength component of 840 nm.

The light source unit 101 includes a light emission device, such as a super luminescent diode (SLD), an LED, a semiconductor optical amplifier (SOA), or the like.

The low coherence light L0 emitted from the light source unit 101 is guided to a fiber coupler 103 through an optical fiber 102. The fiber coupler 103 splits the low coherence light L0 into measurement light LS and reference light LR.

The reference light LR is guided through an optical fiber 104 and arrives at an attenuator (optical attenuator) 105. The attenuator 105 automatically adjusts the amount of the reference light LR having been guided through the optical fiber 104 under the control of the processor 9 using a known technology. The reference light LR whose light amount is adjusted by the attenuator 105 is guided through the optical fiber 104 to arrive at a polarization controller (polarization adjuster) 106. The polarization controller 106 is a device that applies external stress to the looped optical fiber 104 to thereby adjust the polarization state of the reference light LR having been guided through the optical the optical fiber 104. It should be noted that the configuration of the polarization controller 106 is not limited to this and any known technologies can be used. The reference light LR whose polarization state is adjusted by the polarization controller 106 arrives at a fiber coupler 109.

The measurement light LS generated by the fiber coupler 103 is guided to a collimator lens 90 (FIG. 1) through the optical fiber f1, and is made into a parallel light flux by the collimator lens 90. Further, the measurement light LS arrives at the dichroic mirror 83 via an optical path length changing unit 89, an optical scanner 88, the focusing lens 87, the relay lens 85, and the reflective mirror 84.

In some embodiments, the focusing lens 87 and the optical scanner 88 are housed in a single unit that is movable in the optical axis direction. This allows the focusing lens 87 and the optical scanner 88 to move in the optical axis direction, while maintaining the optical positional relationship between the focusing lens 87 and the optical scanner 88. By configuring the focusing lens 87 and the optical scanner 88 in such a way that they can be moved together, it is possible to adjust the optical system while maintaining the conjugate relationship between the optical scanner 88 and the subject's eye E.

In some embodiments, the focusing lens 87 and the optical scanner 88 are moved independently in the unit in the optical axis direction. In some embodiments, the focusing lens 87 and the optical scanner 88 are moved independently or integrally in the optical axis direction under control from the processor 9. For example, the pupil of the subject's eye E is placed at the focal position of the objective lens 51 and the deflection surface of the optical scanner 88 is placed at the focal position of the focusing lens 87. The deflection surface of the optical scanner 88 is disposed at the pupil conjugate position.

The optical path length changing unit 89 changes an optical path length of the measurement light LS. By changing the optical path length of the measurement light LS, a difference between the optical path length of the reference light LR and the optical path length of the measurement light LS can be changed. For example, the optical path length changing unit 89 includes a retroreflector that can move along the optical path of the measurement light LS and the returning light of the measurement light LS, and changes the optical path length of the measurement light LS by moving the retroreflector.

The optical scanner 88 deflects the measurement light LS in a one-dimensionally or two-dimensional manner.

In some embodiments, the optical scanner 88 includes a first galvano mirror and a second galvano mirror. The first galvano mirror deflects the measurement light LS so as to scan the OCT measurement site in the horizontal direction orthogonal to the optical axis of the OCT optical system 8. The second galvano mirror deflects the measurement light LS deflected by the first galvano mirror so as to scan the photographing site in a vertical direction orthogonal to the optical axis of the OCT optical system 8. Examples of scan modes with the measurement light LS performed by the optical scanner 88 like this include horizontal scan, vertical scan, cross scan, radial scan, circle scan, concentric scan, helical (spiral) scan, and the like.

In some embodiments, the optical scanner 88 includes a MEMS scanner (MEMS mirror scanner) that deflects the measurement light LS in a two-dimensional manner. The MEMS scanner deflects the measurement light LS so as to scan the OCT measurement site in the horizontal and vertical directions orthogonal to the optical axis of the OCT optical system 8.

It should be noted that the optical scanner 88 may also include a polygon mirror, a rotating mirror, a dove prism, a double dove prism, a rotation prism, or the like, other than the galvano mirror and the MEMS scanner.

The measurement light LS that has arrived at the dichroic mirror 83 is reflected by the dichroic mirror 83, passes through the relay lens 82, and is reflected by the reflective mirror 81. The measurement light LS reflected by the reflective mirror 81 is reflected by the dichroic mirror 52, is refracted by the objective lens 51, and is irradiated onto the OCT measurement site. The measurement light LS is scattered (and reflected) at various depth positions of the OCT measurement site. Back-scattered light of the measurement light LS from the OCT measurement site reversely advances along the same path as the outward path, and is guided to the fiber coupler 103, and arrives at the fiber coupler 109 through an optical fiber 108.

The fiber coupler 109 causes the back-scattered light of the measurement light LS and the reference light LR, the reference light LR having passed through the attenuator 105, and the like, to interfere with each other. Interference light LC thus generated is guided through an optical fiber 110 and is output from an exit end 111. Further, the interference light LC is collimated into a parallel light flux (beam) by a collimator lens 112, is spectrally divided (spectrally decomposed) by a diffraction grating (spectroscope) 113, is converged by a zoom optical system 114, and is projected onto the light receiving surface of a CCD image sensor 115. Note that although FIG. 2 illustrates the diffraction grating 113 of a transmission type, it is also possible to use a spectrally decomposing element of any other type, such as a diffraction grating of reflection type.

The CCD image sensor 115 is a line sensor, for example, with an array of two or more light receiving elements (detectors). The CCD image sensor 115 detects each spectral component of the dispersed interference light LC, and converts it into electric charge(s). The CCD image sensor 115 accumulates the electric charges to generate a detection signal, and sends the signal to the processor 9.

Although a Michelson interferometer is employed in the present embodiment, it is possible to employ any type of interferometer such as Mach-Zehnder-type as appropriate. In place of the CCD image sensor, an image sensor of other type, such as a complementary metal-oxide semiconductor (CMOS) image sensor, may be used.

Further, the configuration as shown in FIG. 1 is configured to change the difference between the optical path length of the measurement light LS and the optical path length of the reference light LR by changing the optical path length of the measurement light LS by the optical path length changing unit 89. However, the configuration according to the embodiments is not limited to this. For example, by changing the optical path length of the reference light LR using a known method, the configuration may be configured to change the difference between the optical path length of the measurement light LS and the optical path length of the reference light LR.

The processor 9 calculates the refractive power value from the result of the measurement obtained using the refractometry optical system, and controls the refractometry light source 61 and the focusing lens 74 to move respectively to positions where the fundus Ef, the refractometry light source 61, and the imaging element 59 are conjugate with each other, in the optical axis direction based on the calculated refractive power value. In some embodiments, the processor 9 moves the focusing lens 87 and the optical scanner 88 in its optical axis direction in conjunction with the movement of the focusing lens 74. In some embodiments, the processor 9 controls the liquid crystal panel 41 (fixation unit 40) to move in the optical axis direction in conjunction with the movement of the refractometry light source 61 and the focusing lens 74.

In the above embodiments, the function of at least one of the focusing lens 74 or the focusing lens 87 may be realized by a liquid crystal lens or a liquid lens.

< Configuration of processing system >

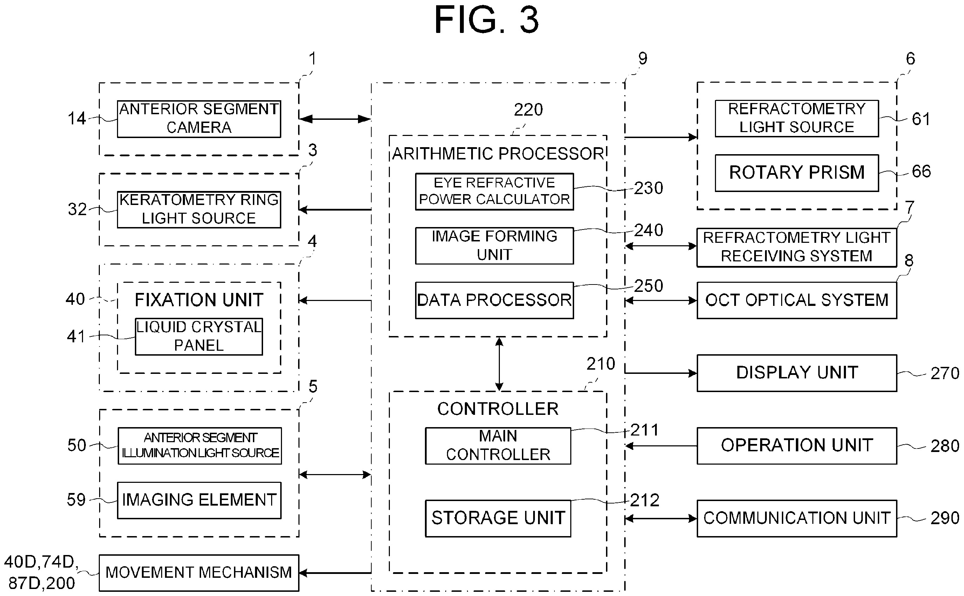

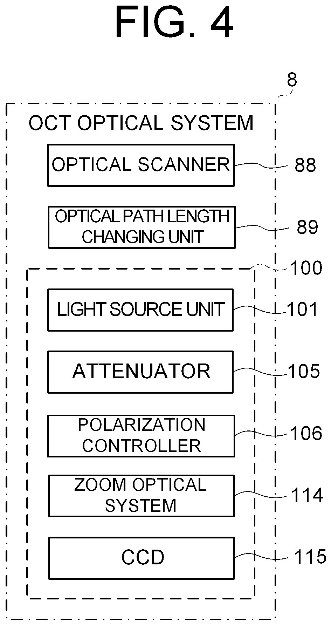

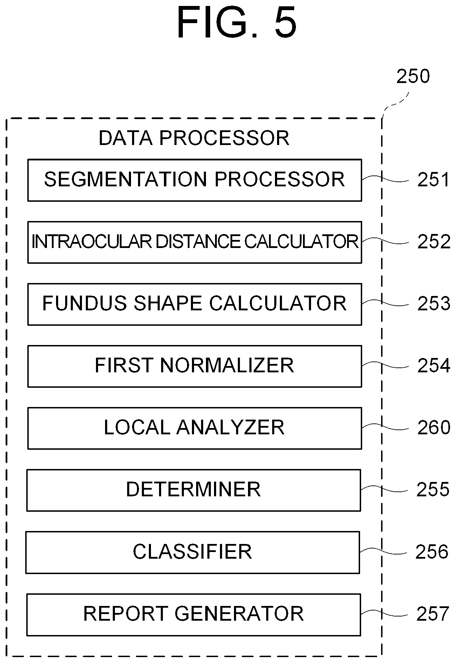

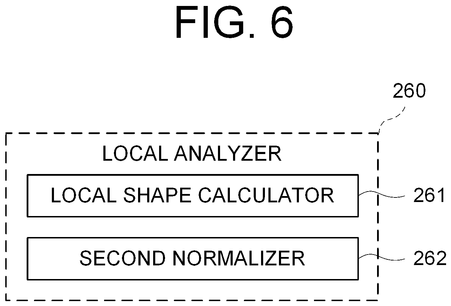

The processing system of the ophthalmic apparatus 1000 will be described. FIGs. 3 to 6 show examples of the functional configuration of the processing system of the ophthalmic apparatus 1000. FIG. 3 shows an example of a functional block diagram illustrating the processing system of the ophthalmic apparatus 1000. FIG. 4 shows an example of a functional block diagram of the OCT optical system 8 in FIG. 3, the OCT optical system 8 being the processing target of the processor 9. FIG. 5 shows an example of a functional block diagram of a data processor 250 in FIG. 3. FIG. 6 shows example of the a functional block diagram of a local analyzer 260 in FIG. 5.

The processor (processing unit) 9 controls each part of the ophthalmic apparatus 1000. Further, the processor 9 is capable of performing various types of arithmetic processing. The processor 9 includes a processor. The processor 9 realizes the function according to the embodiments, for example, by reading out computer program(s) stored in a storage circuit or a storage device and executing the computer program(s).

The processor 9 includes a controller 210 and an arithmetic processor 220. Further, the ophthalmic apparatus 1000 includes movement mechanisms 40D, 74D, 87D, and 200, a display unit 270, an operation unit 280, and a communication unit 290.

The movement mechanism 200 is a mechanism for moving a head unit housing the optical system described above in the X direction, the Y direction, and the Z direction. For example, the movement mechanism 200 is provided with an actuator that generates driving force for moving the head unit and a transmission mechanism that transmits the driving force to the head unit. The actuator is configured by a pulse motor, for example. The transmission mechanism is configured by a combination of gears, a rack and pinion, and the like, for example. The controller 210 (main controller 211) controls the movement mechanism 200 by sending a control signal to the actuator.

The movement mechanism 40D moves the fixation unit 40 in the optical axis direction of the fixation projection system 4 (the optical axis direction of the objective lens 51). For example, the movement mechanism 40D is provided with an actuator that generates a driving force for moving the fixation unit 40 and a transmission mechanism that transmits the driving force from the actuator to the fixation unit 40, similar to the movement mechanism 200. The actuator includes a pulse motor, for example. The transmission mechanism includes a combination of gears, a rack and pinion, and the like, for example. The controller 210 (main controller 211) controls the movement mechanism 40D by sending a control signal to the actuator.

The movement mechanism 74D moves the focusing lens 74 in the optical axis direction of the refractometry light reception system 7. For example, the movement mechanism 74D is provided with an actuator that generates a driving force for moving the focusing lens 74 and a transmission mechanism that transmits the driving force from the actuator to the focusing lens 74, similar to the movement mechanism 200. The actuator includes a pulse motor, for example. The transmission mechanism includes a combination of gears, a rack and pinion, and the like, for example. The controller 210 (main controller 211) controls the movement mechanism 74D by sending a control signal to the actuator.

The movement mechanism 87D moves the focusing lens 87 in the optical axis direction of the OCT optical system 8 (the optical axis direction of the objective lens 51). For example, the movement mechanism 87D is provided with an actuator that generates a driving force for moving the focusing lens 87 and a transmission mechanism that transmits the driving force from the actuator to the focusing lens 87, similar to the movement mechanism 200. The actuator includes a pulse motor, for example. The transmission mechanism includes a combination of gears, a rack and pinion, and the like, for example. The controller 210 (main controller 211) controls the movement mechanism 87D by sending a control signal to the actuator.

In some embodiments, examples of the control for the movement mechanism 87D include control of moving the focusing lens 87 in the optical axis direction, control of moving the focusing lens 87 to the in-focus reference position corresponding to the photographing site, control of moving the focusing lens 87 within the movement range (in-focus range) corresponding to the photographing site, and the like. For example, the main controller 211 controls the movement mechanism 87D by sending a control signal to the actuator to move the focusing lens 87 in the optical axis direction.

( Controller 210 )

The controller 210 includes a processor and controls each part of the ophthalmic apparatus. The controller 210 includes a main controller 211 and a storage unit 212. The storage unit 212 stores, in advance, computer program(s) for controlling the ophthalmic apparatus 1000. Examples of the computer program(s) include a program for controlling the alignment system, a program for controlling the keratometry system, a program for controlling the fixation system, a program for controlling the anterior segment observation system, a program for controlling the refractometry projection system, a program for controlling the refractometry light reception system, a program for controlling the OCT optical system, a program for arithmetic processing, and a program for user interface. The main controller 211 operates according to the computer program(s), and thereby the controller 210 performs the control processing.

The main controller 211 performs various controls of the ophthalmic apparatus, as a measurement controller.

Examples of the control for the alignment system 1 include control of the anterior segment cameras 14.

Examples of the control of the anterior segment cameras 14 include adjustment of exposure of each anterior segment camera, adjustment of gain of each anterior segment camera, adjustment of detection rate of each anterior segment camera, and synchronous control of the photographing of the two anterior segment cameras 14. In some embodiments, the two anterior segment cameras 14 are controlled so that the exposure conditions of the two anterior segment cameras, gains of the two anterior segment cameras, and the detection rates of the two anterior segment cameras are substantially identical.

Examples of control for the keratometry system 3 include control of the keratometry ring light source 32, and the like.

Examples of the control of the keratometry ring light source 32 include turning on and off of the light source, adjustment of a light amount, adjustment of aperture, and the like. Thereby, the keratometry ring light source 32 can be switched between lighting and non-lighting, or the light amount can be changed. The main controller 211 controls the arithmetic processor 220 to perform a known calculation on a keratometry ring image detected by the imaging element 59. Thereby, corneal shape parameter(s) of the subject's eye E is/are obtained.

Examples of the control for the fixation projection system 4 include control of the liquid crystal panel 41, movement control of the fixation unit 40, and the like.

Examples of the control for the liquid crystal panel 41 include displaying on and off of the fixation target, switching the fixation target in accordance with the type of the inspection or the measurement, switching the display position of the fixation target, and the like. The main controller 211 controls the movement mechanism 40D by sending a control signal to the actuator to move at least the liquid crystal panel 41 in the optical axis direction. Thereby, the position of liquid crystal panel 41 is adjusted so that the liquid crystal panel 41 and the fundus Ef are optically conjugate with each other.

Examples of the control for the anterior segment observation system 5 include control of an anterior segment illumination light source 50, control of the imaging element 59, and the like.

Examples of control for the anterior segment illumination light source 50 include turning on and off the light source, adjustment of a light amount, adjustment of aperture, and the like. Thereby, the anterior segment illumination light source 50 is switched between lighting and non-lighting, or the light amount is changed. Example of the control of the imaging element 59 include adjustment of exposure of the imaging element 59, adjustment of gain of the imaging element 59, adjustment of detection rate of the imaging element 59, and the like. The main controller 211 acquires a signal detected by the imaging element 59 and controls the arithmetic processor 220 to perform processing such as forming image based on the acquired signal and the like.

Examples of control for the refractometry projection system 6 include control of the refractometry light source 61, control of the rotary prism 66, and the like.

Examples of the control of the refractometry light source 61 include turning on and off of the light source, adjustment of a light amount, adjustment of aperture, and the like. Thereby, the refractometry light source 61 can be switched between lighting and non-lighting, or the light amount can be changed. For example, the refractometry projection system 6 includes a movement mechanism that moves the refractometry light source 61 in the optical axis direction. As is the case with the movement mechanism 200, each of the movement mechanisms is provided with an actuator that generates driving force for moving the movement mechanism and a transmission mechanism that transmits the driving force from the actuator to the refractometry light source 61. The main controller 211 controls the movement mechanism by sending a control signal to the actuator to move the refractometry light source 61 in the optical axis direction. Examples of the control for the rotary prism 66 include control of rotating the rotary prism 66 and the like. For example, a rotary mechanism that rotates the rotary prism 66 is provided and the main controller 211 controls the rotary mechanism to rotate the rotary prism 66.

The control for the refractometry light reception system 7 includes control of moving the refractometry light source 61 and the focusing lens 74 in the optical axis direction respectively depending on the refractive power of the subject's eye E for example so that the refractometry light source 61 and the fundus Ef and the imaging element 59 are optically conjugate with each other. In this case, the main controller 211 controls the movement mechanism 74D, etc. in accordance with the refractive power of the subject's eye E to arrange the refractometry light source 61, the fundus Ef, and the imaging element 59 at positions optically substantially conjugate with each other.

Examples of the control for the OCT optical system include control of the optical scanner 88, control of the optical path length changing unit 89, control for the OCT unit 100, and the like. Examples of the control for the OCT unit 100 include control of a light source unit 101, control of an attenuator 105, control of a polarization controller 106, control of a zoom optical system 114, control of the CCD image sensor 115, and the like.

Examples of control of the optical scanner 88 include setting the scan mode to scan a measurement site with a predetermined scan pattern, control of a scan range, control of a scan speed, and the like. By controlling the scan range (scan start position and scan end position), an angle range of the deflection surface that deflects the measurement light LS can be controlled. By controlling the scan speed, a change speed of the angle of the deflection surface can be controlled. The main controller 211 controls at least one of the scan mode, the scan range, or the scan speed, by outputting control signal(s) to the optical scanner 88.

Examples of the control of the optical path length changing unit 89 include control of the optical path length of the measurement light LS. The main controller 211 outputs control signal(s) to the optical path length changing unit 89 to change the optical path length of the measurement light LS.

Examples of the control for the light source unit 101 include turning on and off the light source, adjustment of the light amount, adjustment of an aperture, and the like. Examples of the control for the attenuator 105 include adjustment of the light amount of the reference light LR, and the like. Examples of the control for the polarization controller 106 include adjustment of the polarization state of the reference light LR, and the like. Examples of the zoom optical system 114 include control for the optical magnification, and the like. Examples of the control for the CCD image sensor 115 include adjustment of exposure of the CCD image sensor 115, adjustment of gain of the CCD image sensor 115, adjustment of detecting rate of the CCD image sensor 115, and the like. The main controller 211 captures signal(s) detected by the image sensor 115, and controls the arithmetic processor 220 to perform processing such as forming images based on the captured signal(s).

Further, the main controller 211 performs a process of writing data in the storage unit 212 and a process of reading out data from the storage unit 212.

( Storage unit 212 )

The storage unit 212 stores various types of data. Examples of the data stored in the storage unit 212 include a measurement result of the objective measurement (result of OCT measurement), image data of the OCT image, image data of the anterior segment image, a result of the subjective inspection, subject's eye information, and the like. The subject's eye information includes information on the subject such as patient ID and name, and information on the subject's eye such as identification information of the left eye / right eye. Further, the storage unit 212 stores various types of programs and data to run the ophthalmic apparatus.

( Arithmetic processor 220 )

The arithmetic processor 220 includes a processor, and performs various kinds of arithmetic processes. A storage unit not shown (for example, the storage unit 212) stores, in advance, computer program(s) for performing various kinds of arithmetic processes. The processor operates according to the computer program(s), and thereby the processor realizes the functions of each part that performs the various kinds of arithmetic processes.

As shown in FIG. 3, the arithmetic processor 220 includes an eye refractive power calculator 230, an image forming unit 240, and a data processor 250. The refraction power calculator 230 calculates a refractive power (dioptric power) of the subject's eye E. The image forming unit 240 forms the OCT image data based on the detection result of the interference light LC acquired using the OCT optical system 8. The data processor 250 performs various kinds of data processing (e.g., image processing) and various kinds of analysis on the measurement result (detection result of the interference light LC, etc.) obtained using the optical system included in the ophthalmic apparatus 1000 and/or the OCT image formed by the image forming unit 240.

( Eye refractive power calculator 230 )

The eye refractive power calculator 230 analyzes a ring image (pattern image) acquired by receiving the returning light of the ring-shaped light flux (ring-shaped measurement pattern) by the imaging element 59, the ring-shaped light flux being projected onto the fundus Ef by the refractometry projection system 6. For example, the eye refractive power calculator 230 obtains a position of the center of gravity of the ring image from the brightness distribution in the image representing the acquired ring image, obtains brightness distributions along a plurality of scanning directions extending radially from the position of the center of gravity, and identifies a ring image from these brightness distributions. Subsequently, the eye refractive power calculator 230 obtains an approximate ellipse of the identified ring image and obtains a refractive power (spherical power, an astigmatic power, and an astigmatic axis angle) by assigning a major axis and a minor axis of the approximate ellipse to a known formula. Alternatively, the eye refractive power calculator 230 can obtain parameter (dioptric power) of the eye refractive power based on deformation and displacement of the ring image with reference to the reference pattern.

Further, the eye refractive power calculator 230 calculates a corneal refractive power, a corneal astigmatism power, and a corneal astigmatic axis angle based on the keratometry ring image acquired by the anterior segment observation system 5. For example, the eye refractive power calculator 230 calculates a corneal curvature radius of the steepest meridian and/or the flattest meridian of the anterior surface of the cornea by analyzing the keratometry ring image and calculates above parameters based on the corneal curvature radius.

( Image forming unit 240 )

The image forming unit 240 forms the image data of the OCT image of the subject's eye E based on the detection result of the interference light LC acquired using the CCD image sensor 115. In other words, the image forming unit 240 forms the image data of the subject's eye E based on the detection result of the interference light LC obtained by the interference optical system. Like the conventional spectral-domain-type OCT, this process includes processes such as filtering and FFT (Fast Fourier Transform). The image data acquired in this manner is a data set including a group of image data formed by imaging the reflection intensity profiles of a plurality of A lines. Here, the A lines are the paths of the measurement light LS in the subject's eye E.

In order to improve the image quality, it is possible to repeatedly perform scan with the same pattern a plurality of times to acquire a plurality of data sets, and to compose (i.e., average) the plurality of data sets.

( Data processor 250 )

The data processor 250 performs various types of data processing (image processing) and various types of analysis on an OCT image formed by the image forming unit 240 or a detection signal of the interference light LC obtained by the image sensor 115. For example, the data processor 250 performs various correction processes such as brightness correction and dispersion correction of images. Further, the data processor 250 performs various types of image processing and analysis on images (anterior segment image, etc.) acquired using the anterior segment observation system 5.

The data processor 250 can perform known image processing such as interpolation for interpolating pixels between the OCT images (tomographic image) formed by the image forming unit 240 to form the image data of the three-dimensional image of the fundus Ef or the anterior segment. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. To display an image based on volume data, the data processor 250 performs image rendering processing (e.g., volume rendering, maximum intensity projection (MIP)) on the volume data to form image data of a pseudo three-dimensional image taken from a specific view direction. The pseudo three-dimensional image is displayed on a display device such as the display unit 270.

Further, the three-dimensional image data may be stack data of a plurality of tomographic images. The stack data is image data formed by three-dimensionally arranging tomographic images along a plurality of scan lines based on positional relationship of the scan lines.

That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 250 can form a B-mode image (longitudinal cross-sectional image, axial cross-sectional image) in an arbitrary cross section, a C-mode image (transverse section image, horizontal cross-sectional image) in an arbitrary cross section, a projection image, a shadowgram, etc., by performing various renderings on the acquired three-dimensional data set (volume data, stack data, etc.). An image in an arbitrary cross section such as a B-mode image or a C-mode image is formed by selecting pixels (voxels) on a designated cross section from the three-dimensional data set. The projection image is formed by projecting the three-dimensional data set in a predetermined direction (Z direction, depth direction, axial direction). The shadowgram is formed by projecting a part of the three-dimensional data set in a predetermined direction. Examples of the part of the three-dimensional data set include partial data corresponding to a specific layer. An image having a viewpoint on the front side of the subject's eye, such as the C-mode image, the projection image, and the shadowgram, is called a front image (en-face image).

The data processor 250 can build (form) the B-mode image or the front image (blood vessel emphasized image, angiogram) in which retinal blood vessels and choroidal blood vessels are emphasized (highlighted), based on data (for example, B-scan image data) acquired in time series by performing OCT scan. For example, the OCT data in time series can be acquired by repeatedly scanning substantially the same site of the subject's eye E.

In some embodiments, the data processor 250 compares the B-scan images in time series acquired by B-scan for substantially the same site, converts the pixel value of a change portion of the signal intensity into a pixel value corresponding to the change portion, and builds the emphasized image in which the change portion is emphasized. Further, the data processor 250 forms an OCTA image by extracting information of a predetermined thickness at a desired site from a plurality of built emphasized images and building as an en-face image.

An image (for example, a three-dimensional image, a B-mode image, a C-mode image, a projection image, a shadowgram, and an OCTA image) generated by the data processor 250 is also included in the OCT image.

Further, the data processor 250 identifies the characteristic position corresponding to the characteristic site of the anterior segment, by analyzing each of the two photographic images substantially simultaneously obtained using the two anterior segment cameras 14. The characteristic site of the anterior segment is, for example, a center (center of gravity) of the pupil. The data processor 250 calculates the three-dimensional position of the characteristic site (i.e., three-dimensional position of the subject's eye E) by applying a known trigonometry to the positions of the two anterior segment cameras 14 and the identified characteristic position corresponding to the characteristic site in the two photographic images. The calculated three-dimensional position can be used for the position matching of the optical system with respect to the subject's eye E.

Further, the data processor 250 can perform predetermined analysis processing on the OCT data, the OCT images, or the fundus images. Examples of the predetermined analysis processing include identifying of a predetermined site (tissue, site of lesion) of the subject's eye E, calculating a distance between designated sites (distance between layers, interlayer distance), area, angle, ratio, or density; calculating by a designated formula; identifying the shape of a predetermined site; calculating these statistics; calculating distribution of the measured value or the statistics; image processing based on these analysis processing results, and the like.

Examples of the predetermined site include a blood vessel, a site of lesion, and the like. Examples of the site of lesion include a detachment part, a hydrops, a hemorrhage, a tumor, and a drusen.

The data processor 250 can, for example, identify a predetermined site or a predetermined layer region, identify a cross-sectional position with reference to the identified predetermined site or the identified layer region, and form the tomographic image at a desired cross-sectional position by selecting or interpolating pixels in a desired cross-sectional orientation at the desired cross-sectional position, in the three-dimensional OCT data. It should be noted that in this processing of generating the tomographic image, a tomographic image in the desired cross-sectional orientation at a cross-sectional position designated by the user using an operation unit 280 described below may be generated.

Further, the data processor 250 can calculate shape data representing the shape of the fundus Ef or the intraocular distance in the fundus Ef, based on the two-dimensional or three-dimensional OCT data (measurement data) obtained by performing OCT scan on the subject's eye E. Furthermore, the data processor 250 can calculate the normalized shape data or normalized intraocular distance by normalizing the calculated shape data or the calculated intraocular distance using the axial length of the subject's eye E as the body data. The axial length can be calculated from one-dimensional, two-dimensional, or three-dimensional OCT data. In some embodiments, the data processor 250 calculates the normalized shape data or the normalized intraocular distance by scaling (normalizing) the OCT data using the axial length and calculating the shape data representing the shape of the fundus Ef or the intraocular distance from the scaled OCT data.

The data processor 250 having such a configuration includes, as shown in FIG. 5, a segmentation processor 251, an intraocular distance calculator 252, a fundus shape calculator 253, a first normalizer 254, a local analyzer 260, a determiner 255, a classifier 256, and a report generator 257. The local analyzer 260 includes, as shown in FIG. 6, a local shape calculator 261 and a second normalizer 262.

( Segmentation processor 251 )

The segmentation processor 251 identifies the predetermined layer region in the anterior segment or the fundus Ef based on the OCT data. Examples of the predetermined layer region include the inner limiting membrane, the nerve fiber layer, the ganglion cell layer, the inner plexiform layer, the inner nuclear layer, the outer plexiform layer, the outer nuclear layer, the external limiting membrane, the photoreceptor layer, the RPE layer, the Bruch membrane, the choroid, the sclera, the vitreous body, or the like.

For example, the segmentation processor 251 analyzes the one-dimensional, two-dimensional or three-dimensional OCT data to identify a plurality of layer regions in the A-scan direction. For example, the segmentation processor 251 obtains the gradients of the pixel values (i.e., brightness values) in each A scan image included in the OCT data, and identifies a position where the gradient value is large to be a tissue boundary. The segmentation processor 251 identifies, for example, a layer tissue for a predetermined number of pixels on the sclera side with respect to the identified RPE layer as the Bruch membrane. It should be noted that the A-scan image is one-dimensional image data extending in the depth direction of the fundus. The depth direction of the fundus is defined as, for example, the Z direction, the incident direction of the OCT measurement light, the axial direction, the optical axis direction of the interference optical system, or the like.

( Intraocular distance calculator 252 )

The intraocular distance calculator 252 calculates the intraocular distance of the subject's eye E by analyzing the one-dimensional, two-dimensional, or three-dimensional OCT data. Examples of the intraocular distance include a distance in the Z direction and an intraocular distance in the XY directions. Examples of the intraocular distance in the Z direction include the axial length and a distance (thickness) between predetermined layer regions in the cornea or the fundus Ef. Examples of the intraocular distance in the XY directions include a distance between predetermined sites in the anterior segment (e.g., distance from a corneal apex to a predetermined position on the anterior surface of cornea), and a distance between predetermined sites in the fundus Ef (e.g., distance between a macula and an optic disc).

In case of calculating the intraocular distance in the XY directions, the intraocular distance calculator 252 calculates the distance between two desired sites identified by the data processor 250 based on the OCT data, the OCT image, the anterior segment image, or the fundus image. In case of calculating the intraocular distance in the Z direction, the intraocular distance calculator 252 calculates the distance between the two desired layer regions (sites) identified by the segmentation processor 251 based on the OCT data or the OCT image.

( Fundus shape calculator 253 )

The fundus shape calculator 253 calculates the shape data representing the shape of the fundus Ef by analyzing the two-dimensional or three-dimensional OCT data. For example, the fundus shape calculator 253 identifies a shape profile (depth profile) representing a change in depth positions (Z positions) of the fundus Ef in a predetermined range in a direction orthogonal (intersecting) the Z direction from the OCT data, and calculates the radius of curvature (or curvature) of the fundus Ef as the shape data of the fundus Ef based on the identified shape profile.

In some embodiments, the fundus shape calculator 253 obtains a circle (an ellipse) through a plurality of points on the identified shape profile using a known method, and calculates the radius of curvature (or curvature) of the fundus Ef based on the obtained circle (ellipse). In some embodiments, the fundus shape calculator 253 performs circle fitting (ellipse fitting) on the identified shape profile, and calculates the radius of curvature (or curvature) of the fundus Ef based on the obtained approximate circle (approximate ellipse). In some embodiments, the fundus shape calculator 253 performs polynomial fitting on the identified shape profile, and calculates the radius of curvature (or curvature) of the fundus Ef based on the obtained approximate polynomial.

In the present embodiment, the fundus shape calculator 253 calculates the shape data representing the shape of the RPE layer. In other words, the fundus shape calculator 253 identifies the shape profile of the RPE layer from the OCT data, and calculates the radius of curvature (or curvature) of the RPE layer as the shape data of the fundus Ef based on the identified shape profile.

( First normalizer 254 )

The first normalizer 254 normalizes the shape data representing the shape of the fundus Ef calculated by the fundus shape calculator 253 or the intraocular distance in the XY directions calculated by the intraocular distance calculator 252, based on the body data of the subject or the refractive power of the subject's eye E. The refractive power of the subject's eye E may be the refractive power calculated by the eye refractive power calculator 230 or a refractive power measured using an external ophthalmic apparatus.

The first normalizer 254 normalizes the radius of curvature r (or curvature) as the shape data of the fundus Ef based on the axial length AL as the body data of the subject according to Equation (1) to calculate a normalized radius of curvature r' (normalized shape data).

[Equation 1]

$$r' = \frac{2r}{AL} \quad \cdots (1)$$

In some embodiments, the first normalizer 254 normalizes the radius of curvature r (or curvature) as the shape data of the fundus Ef based on the axial length AL as the body data of the subject according to Equation (2) to calculate a normalized radius of curvature r' (normalized shape data).

[Equation 2]

$$r' = \frac{AL}{2r} \quad \cdots (2)$$

The first normalizer 254 can normalize the shape data of the fundus Ef based on the body data other than the axial length or the refractive power of the subject's eye E, as in Equation (1) or Equation (2). In Equations (1) and (2), the multiplier of the radius of curvature r may be other than "2".

( Local analyzer 260 )

The local analyzer 260 locally analyzes the shape of the fundus Ef. Specifically, the local analyzer 260 normalizes the shape data representing a local shape of the fundus Ef (or the intraocular distance of the subject's eye E) based on the body data of the subject or the refractive power of the subject's eye E to obtain normalized local shape data. For example, when it is determined, based on the normalized shape data or the normalized intraocular distance obtained by the first normalizer 254, that the shape, etc. of the fundus Ef needs to be analyzed in detail, detailed analysis can be performed using the normalized local shape data, etc. obtained by the local analyzer 260.

( Local shape calculator 261 )

The fundus shape calculator 253 described above can calculate the radius of curvature (or curvature) of the fundus Ef from a single shape profile in a predetermined range in the direction(s) orthogonal to the Z direction. In contrast, the local shape calculator 261 can calculate a plurality of radii of curvature (or curvatures) of the fundus Ef for each range, from each of a plurality of shape profiles in a plurality of ranges obtained by dividing the predetermined range in the direction orthogonal to the Z direction.

The local curvature (or radius of curvature) in each range can be calculated in the same way as in the fundus shape calculator 253.

In the present embodiment, the local shape calculator 261 identifies the local shape profile of the RPE layer from the OCT data, and calculates the local radius of curvature (or curvature) of the RPE layer based on the identified local shape profile. This allows the local shape calculator 261 to calculate the plurality of radii of curvature (or curvatures) of the RPE layer in the predetermined range by calculating the local radius of curvature (or curvature) of the RPE layer for each range.

( Second normalizer 262 )

The second normalizer 262 normalizes the shape data representing the local shape of the fundus Ef calculated by the local shape calculator 261 or the local intraocular distance in the XY directions calculated by the intraocular distance calculator 252, based on the body data of the subject or the refractive power of the subject's eye E.

As in the first normalizer 254, the second normalizer 262 normalizes the radius of curvature r (or curvature) as the shape data of the fundus Ef based on the axial length AL as the body data of the subject according to Equation (1) or Equation (2) to calculate the normalized radius of curvature r' (normalized shape data).

( Determiner 255 )

The determiner 255 determines whether or not the eye shape of the subject's eye E is abnormal (normal) based on the shape data of the subject's eye E normalized by the first normalizer 254 (or the shape data of the fundus Ef calculated from the normalized measurement data). For example, the determiner 255 determines whether or not the eye shape of the subject's eye E is abnormal, by comparing the normalized shape data of the fundus Ef with normative data (Normative Data) that indicates the relationship between the shape data of the fundus and the abnormality of the eye shape. It should be noted that the normative data is generated by investigating the relationship between the shape data of the fundus and the abnormality of the eye shape for a plurality of eyes of the subjects in advance.

In some embodiments, the determiner 255 determines whether the eye shape of the subject's eye E is abnormal based on time-series data of the normalized shape data of the fundus Ef. For example, the determiner 255 determines whether or not the eye shape of the subject's eye E is abnormal, by comparing the time-series data of the shape data of the fundus with normative data that indicates the relationship between the time-series data of the shape data of the fundus and the abnormality of the eye shape.

The controller 210 can notify the determination result obtained by the determiner 255, as a notifier. For example, when it is determined by the determiner 255 based on the normalized shape data of the fundus Ef that the eye shape of the subject's eye E is abnormal, the controller 210 notifies information indicating that the eye shape of the subject's eye E is determined to be abnormal. For example, when it is determined by the determiner 255 based on the time-series data of the normalized shape data of the fundus Ef that the eye shape of the subject's eye E is abnormal or is approaching abnormality, the controller 210 notifies information indicating that the eye shape of the subject's eye E is determined to be abnormal. Examples of the notification include image display on the display unit 270, sound output, light output, etc.

( Classifier 256 )

The classifier 256 classifies the eye shape (or change in the eye shape) of the subject's eye E into any of a plurality of eye shape types determined in advance, based on the shape data (or intraocular distance) normalized by the first normalizer 254 or the second normalizer 262. The classifier 256 can perform the classification described above using the shape data, etc. normalized by the first normalizer 254 as a single indicator. The classifier 256 can perform the classification described above using statistics of the plurality of shape data, etc. normalized by the second normalizer 262 as a single indicator. Examples of the statistics include an average value, a minimum value, a maximum value, a median, a mode, a standard deviation, a variance, and an average value.

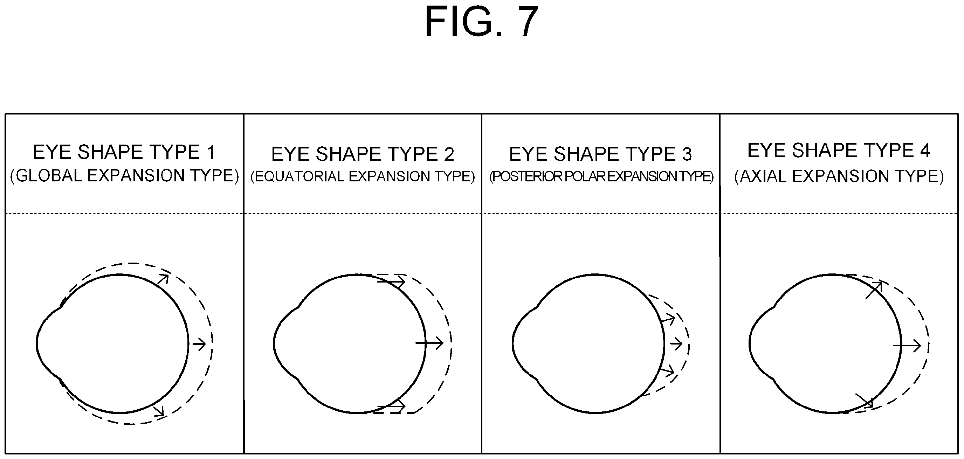

FIG. 7 shows a diagram for explaining the eye shape types according to the embodiments. FIG. 7 schematically represents four eye shape types of the eye shape (see Non-Patent Document 1).

The eye shape type 1 is a Global expansion type, in which the eye shape changes so as to expand uniformly over a wide range including an equatorial part and a posterior segment of the eye. The eye shape type 2 is an Equatorial expansion type, in which the eye shape changes so that the equatorial part of the eye expands parallel to a direction of an ocular axis. The eye shape type 3 is a Posterior polar expansion type, in which an area near the posterior pole in the fundus of the eye expands in the direction of the ocular axis. The eye shape type 4 is an Axial expansion type, in which the fundus of the eye expands in the direction of the ocular axis. The eye shape type 3 and the eye shape type 4 can be defined as the same eye shape type.

The classifier 256 classifies the eye shape of the subject's eye E into one of four types of the eye shape type 1 to the eye shape type 4, based on the shape data (or intraocular distance) normalized by the first normalizer 254 or the second normalizer 262. In some embodiments, the classifier 256 classifies the eye shape of the subject's eye E into one of three types including the eye shape type 1, the eye shape type 2, and the eye shape type 3 (or the eye shape type 4), based on the normalized shape data, etc.

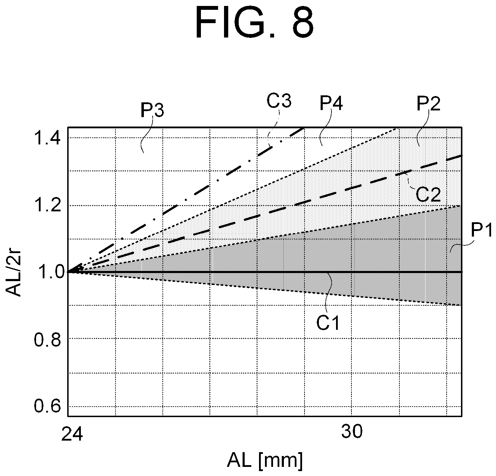

FIG. 8 schematically shows the relationship between the shape data normalized using the axial length by the first normalizer 254 and the axial length. In FIG. 8, the horizontal axis represents the axial length [mm], and the vertical axis represents the radius of curvature (= AL / 2r) of the fundus Ef normalized using the axial length.

As shown in FIG. 8, for the eye shape type 1, the normalized shape data (= AL / 2r) changes according to a characteristic line C1 with respect to the axial length. And, for the eye shape type 2, the normalized shape data changes according to a characteristic line C2 with respect to the axial length. Therefore, when the normalized shape data is included in the characteristic line C1 or a range P1 including the characteristic line C1, the classifier 256 can classify the eye shape of the subject's eye into the eye shape type 1. Here, the range P1 is a range that includes the characteristic line C1 and is surrounded by a dashed line, and can be changed arbitrarily. In the same way, when the normalized shape data is included in the characteristic line C2 or a range P2 including the characteristic line C2, the classifier 256 can classify the eye shape of the subject's eye into the eye shape type 2. Here, the range P2 is a range that includes the characteristic line C2 and is surrounded by a dashed line, and can be changed arbitrarily.

The eye shape type 3 is thought to tend to have larger normalized shape data than the eye shape type 4. Thus, for example, when the characteristic line C3 for distinguishing between the eye shape type 3 and the eye shape type 4 is determined based on the normative data, the classifier 256 can classify the eye shape of the subject's eye E into the eye shape type 3 or the eye shape type 4 by determining whether the normalized shape data is includes in a range upward from the characteristic line C3 or a range downward from the characteristic line C3. Specifically, when the normalized shape data is included in the range P3 upward from the characteristic line C3, the classifier 256 classifies the eye shape of the subject's eye E into the eye shape type 3. And, when the normalized shape data is included in the range P4 downward from the characteristic line C3, the classifier 256 classifies the eye shape of the subject's eye E into the eye shape type 4.

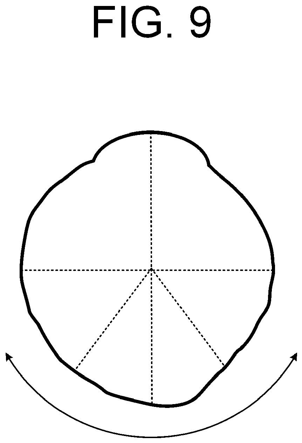

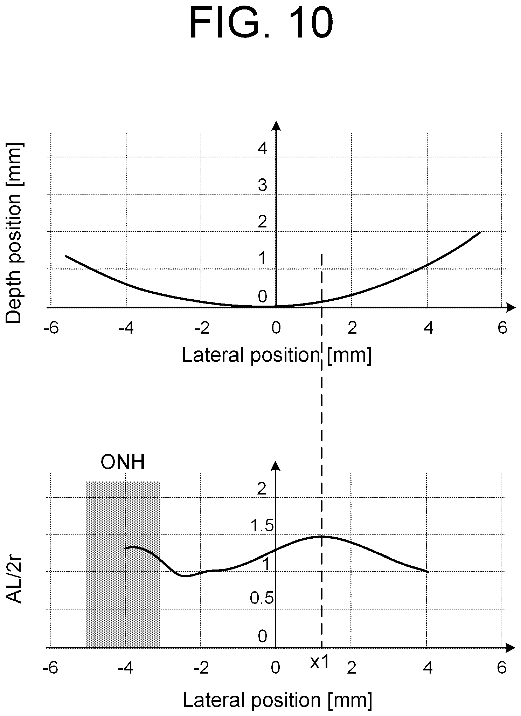

FIG. 9 and FIG. 10 show diagrams for explaining the local analyzer 260. FIG. 9 schematically represents an example of a shape of an actual eye. FIG. 10 represents an example of the normalized local shape data obtained by the local analyzer 260. In FIG. 10, the upper graph schematically represents the shape profile of the RPE layer in which the horizontal axis represents lateral (horizontal) position [mm] and the vertical axis representing the depth position [mm], and the lower graph schematically represents the profile of the local shape data normalized using the axial length by the second normalizer 262. In the lower graph in FIG. 10, the horizontal axis represents the lateral position [mm] and the vertical axis represents the radius of curvature (= AL / 2r) of the fundus Ef normalized using the axial length.

As shown in FIG. 9, the eye shape may have an asymmetric shape relative to the visual axis (or measurement axis). In this case, as shown in FIG. 10, a shape profile similar to that of an eye with a symmetrical shape relative to the visual axis may be obtained. In contrast, by normalizing the local shape data by the second normalizer 262, it can be seen that a change in shape that is due to factors other than growth factors at the shifted position x1 relative to the visual axis should be focused on.

In this case, the classifier 256 can determine whether or not the shape of the fundus Ef is the eye shape type 3 with reference to the maximum value of the local shape data normalized by the second normalizer 262. For example, when the maximum value of the local shape data normalized by the second normalizer 262 is "1.5" or greater, the classifier 256 classifies the shape of the fundus Ef into the eye shape type 3.

Such classifier 256 can classify the eye shape of the subject's eye E into any of the four types including the eye shape type 1 to the eye shape type 4, based on the local shape data normalized by the second normalizer 262.

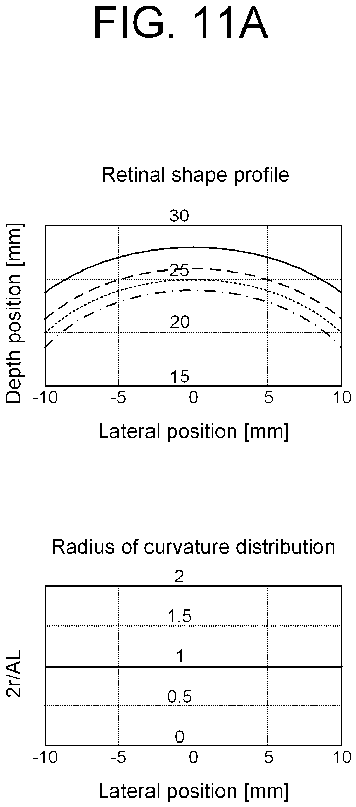

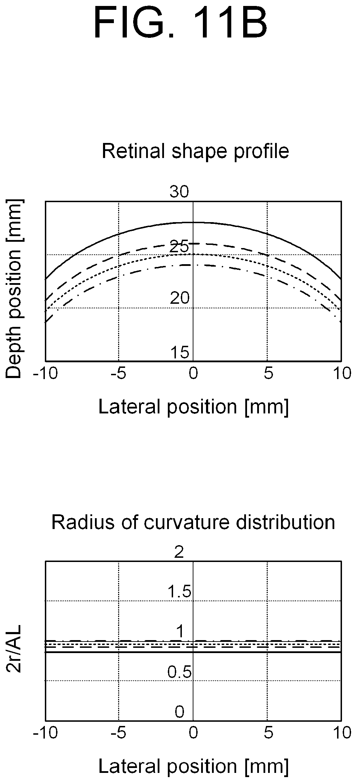

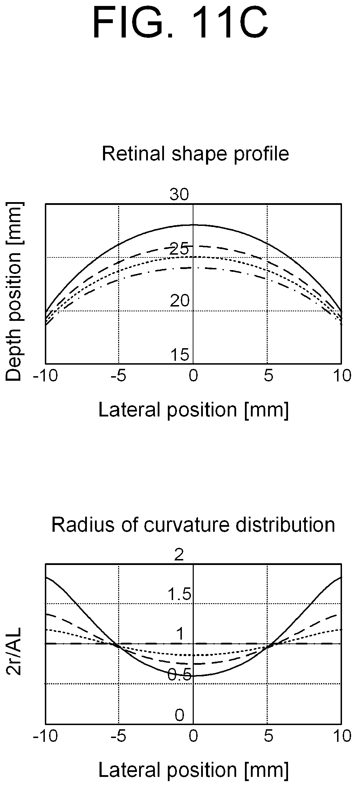

FIGs. 11A to 11C schematically show the relationship between the shape profiles of eye shape type 1, the eye shape type 2, and the eye shape type 4 in FIG. 7 and the local shape data normalized by the second normalizer 262. FIG. 11A represents the relationship between the shape profile in the eye shape type 1 and the local shape data normalized by the second normalizer 262. FIG. 11B represents the relationship between the shape profile in the eye shape type 2 and the local shape data normalized by the second normalizer 262. FIG. 11C represents the relationship between the shape profile in the eye shape type 4 and the local shape data normalized by the second normalizer 262. The shape profiles in FIGs. 11A to 11C represents the shape profiles of the RPE layer in which the horizontal axis represents the lateral (horizontal) position [mm] and the vertical axis represents the depth position [mm]. In the profiles of the normalized local shape data in FIGs. 11A to 11C, the horizontal axis represents the lateral position [mm] and the vertical axis represents the normalized shape data (= 2r / AL). It should be noted that the normalized shape data shown in FIGs. 11A to 11C are the inverse of the normalized shape data shown in FIG. 8 or FIG. 10.

In the eye shape type 1 shown in FIG. 11A, the normalized shape data (=2r / AL) is approximately "1" for a plurality of shape profiles with different axial lengths. In the eye shape type 2 shown in FIG. 11B, the normalized shape data is a value shifted from "1" for a plurality of shape profiles with different axial lengths. In the eye shape type 4 shown in FIG. 11C, the normalized shape data differs significantly depending on the lateral position for a plurality of shape profiles with different axial lengths.

Therefore, the eye shape type can be identified from the absolute value of the normalized radius of curvature distribution and the shape of the radius of curvature distribution. For example, when the normalized radius of curvature distribution is uniform (discriminable based on standard deviation and/or variance) for changes in lateral position, the classifier 256 classifies the eye shape into the eye shape type 1 or the eye shape type 2. Furthermore, when the average value of the normalized radius of curvature distribution for changes in lateral position is within a predetermined range, the classifier 256 classifies the eye shape into the eye shape type 1. And, when the average value exceeds the predetermined range, the classifier 256 classifies the eye shape into the eye shape type 2. In contrast, when the normalized radius of curvature distribution is not uniform for changes in lateral position, the classifier 256 classifies the eye shape into the eye shape type 4.

(Report generator 257 )

The report generator 257 generates a report representing the results of data processing performed by the data processor 250. Examples of the report generated by the report generator 257 include an image in which a predetermined layer region identified by the segmentation processor 251 in the tomographic image formed by the image forming unit 240 is distinguishable, an intraocular distance calculated by the intraocular distance calculator 252, shape data normalized by the first normalizer 254, local shape data obtained by the local analyzer 260, local shape data normalized by the second normalizer 262, determination result(s) obtained by the determiner 255, classification result(s) obtained by the classifier 256. In some embodiments, the report generator 257 generates a report including time-series data of the normalized shape data or time-series data of the normalized local shape data. For example, the controller 210 causes the display unit 270 to display the report generated by the report generator 257, as a display controller. In other words, the controller 210 causes the display unit 270 to display the time-series data of the normalized shape data (or intraocular distance) or the normalized local shape data (or intraocular distance) with different measurement timing from each other.

The functions of the data processor 250 are realized by one or more processors. In some embodiments, the data processor 250 includes two or more processors corresponding to each part of the data processor 250 described above, and is configured so that each processor realizes a function of each part of the data processor 250.

( Display unit 270, Operation unit 280 )

Upon receiving control of the controller 210, the display unit 270 displays information, as a user interface unit. The display unit 270 includes the display unit 10 as illustrated in FIG. 1 and the like.

The operation unit 280 is used to operate the ophthalmic apparatus, as the user interface unit. The operation unit 280 includes various types of hardware keys (the joystick, buttons, switches, etc.) provided in the ophthalmic apparatus. Further, the operation unit 280 may include various kinds of software keys (buttons, icons, menus, etc.) displayed on the touch panel type display screen.

At least part of the display unit 270 and the operation unit 280 may be integrally configured. A typical example of this is the touch-panel display screen 10a.

( Communication unit 290 )

The communication unit 290 has the function of communicating with an external device (not shown). The communication unit 290 includes a communication interface according to the mode of communication with the external device. Examples of the external device includes an eyeglass lens measurement device for measuring the optical properties of lenses. The eyeglass lens measurement device measures the power of the eyeglass lens worn by the subject and inputs the measurement data to the ophthalmic apparatus 1000. The external device may also be a device (reader) having the function of reading information from a recording medium or a device (writer) having the function of writing information to a recording medium. Further, the external device may be a hospital information system (HIS) server, a Digital Imaging and Communications in Medicine (DICOM) server, a doctor terminal, a mobile terminal, a personal terminal, a cloud server, or the like. The communication unit 290 may be provided in the processor 9, for example.

The ophthalmic apparatus 1000, or the data processor 250 and the communication unit 290 is an example of the "ophthalmic information processing apparatus" according to the embodiments. The optical system (particularly, OCT optical system 8) shown in FIG. 1 and FIG. 2 or the communication unit 290 is an example of the "acquisition unit" according to the embodiments. The first normalizer 254 or the second normalizer 262 is an example of the "normalizer" according to the embodiments. The fundus shape calculator 253 or the local shape calculator 261 is an example of the "calculator" according to the embodiments. The controller 210 (main controller 211) is an example of the "display controller" according to the embodiments. The controller 210 (main controller 211) is an example of the "notifier" according to the embodiments. The optical system (particularly, OCT optical system 8) shown in FIG. 1 and FIG. 2 is an example of the "measurement system" according to the embodiments.

<Operation example>

Some examples of the operation of the ophthalmic apparatus 1000 according to the embodiments will be described.

[ First operation example ]

A first operation example is an operation example when the normalized shape data of the fundus Ef (specifically, fundus curvature) is used as a single indicator to classify the eye shape type of the subject's eye E.

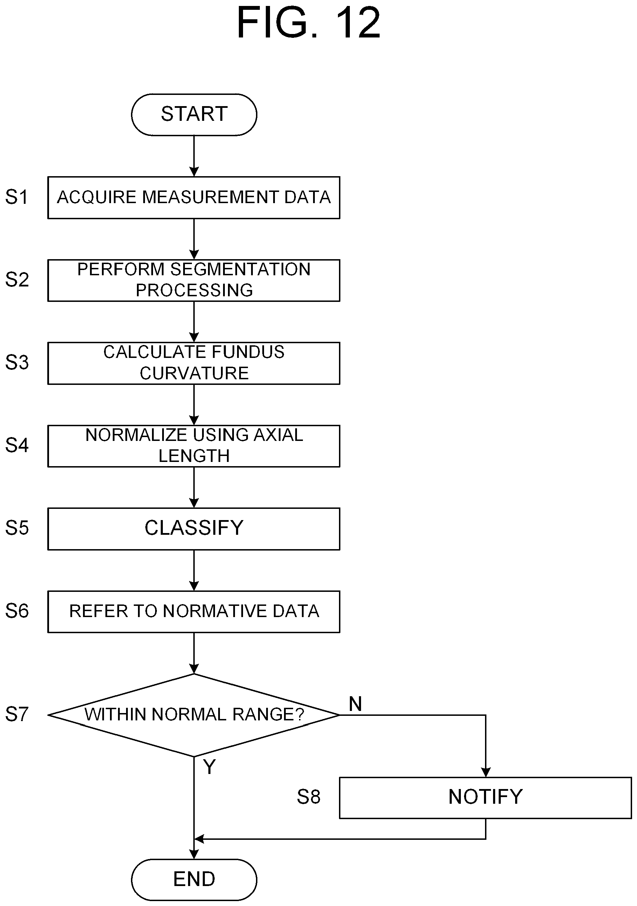

FIG. 12 shows the first operation example of the ophthalmic apparatus 1000. FIG. 12 represents a flowchart of the first operation example of the ophthalmic apparatus 1000. The storage unit 212 stores computer program(s) for realizing the processing shown in FIG. 12. The main controller 211 operates according to the computer program(s), and thereby the main controller 211 performs the processing shown in FIG. 12. In the following, it is assumed that position matching of the optical system relative to the subject's eye E has already been completed. Further, it is assumed that the storage unit 212 stores the normative data indicating the relationship between the fundus curvatures normalized using the axial lengths and the abnormal ranges (normal ranges) of the eye shape.

( S1: Acquire measurement data)

First, the main controller 211 controls the OCT optical system 8 to perform OCT scan on the scan range set on the fundus Ef of the subject's eye E. The detection results of the interference light LC obtained by performing OCT scan are imaged by the image forming unit 240 and the two-dimensional or three-dimensional OCT data is generated as the measurement data by the data processor 250.

( S2: Perform segmentation processing )

Next, the main controller 211 controls the segmentation processor 251 to perform segmentation processing on the measurement data acquired in step S1. The segmentation processor 251 identifies the RPE layer.

( S3: Calculate fundus curvature)

Next, the main controller 211 controls the fundus shape calculator 253 to calculate the curvature of the RPE layer as the fundus curvature from the shape profile of the RPE layer obtained in step S2. It should be noted that the fundus shape calculator 253 may calculate the radius of curvature of the RPE layer from the shape profile of the RPE layer.

( S4: Normalize using axial length )

Next, the main controller 211 controls the first normalizer 254 to normalize the fundus curvature calculated in step S3, using the axial length of the subject's eye E. The first normalizer 254 normalizes the fundus curvature as shown in Equation (1) or Equation (2).

It should be noted that the axial length of the subject's eye E may be calculated by the intraocular distance calculator 252 from the measurement data acquired in step S1. In some embodiments, the axial length of the subject's eye E is obtained from an external ophthalmic apparatus or external server by means of the communication unit 290.

( S5: Classify)

Subsequently, the main controller 211 controls the classifier 256 to classify the eye shape of the subject's eye E into any of the eye shape type 1 to the eye shape type 4 shown in FIG. 7.

For example, the classifier 256 identifies the eye shape type of the subject's eye E by determining whether the fundus curvature normalized using the axial length in step S4 falls within any of the ranges P1 to P4 shown in FIG. 8.

( S6: Refer to normative data)

Next, the main controller 211 controls the determiner 255 to refer to the normative data stored in the storage unit 212. For example, in the normative data, the normalized fundus curvature normalized using the axial length in the range of normal (or abnormal) eye shape is stored.

( S7: Within normal range? )

Subsequently, the main controller 211 controls the determiner 255 to determine whether or not the eye shape of the subject's eye is within the normal range by comparing the normative data referred in step S6 with the normalized fundus curvature acquired in step S4.

When it is determined in step S7 that the eye shape of the subject's eye E is within normal range (S7: Y), the ophthalmic apparatus 1000 terminates the operation (END). When it is determined in step S7 that the eye shape of the subject's eye E is not within the normal range (S7: N), the operation of the ophthalmic apparatus 1000 proceeds to step S8.

( S8: Notify )

When it is determined in step S7 that the eye shape of the subject's eye E is not within the normal range (S7: N), the main controller 211 notifies the information indicating that the eye shape of the subject's eye E is determined to be abnormal. Examples of the notification include image display on the display unit 270, sound output, light output, report output, etc. This terminates the first operation example of the ophthalmic apparatus 1000 (END).

[ Second operation example ]

A second operation example is an operation example when the normalized shape data of the fundus Ef is used as a single indicator to analyze the eye shape type of the subject's eye E in detail.

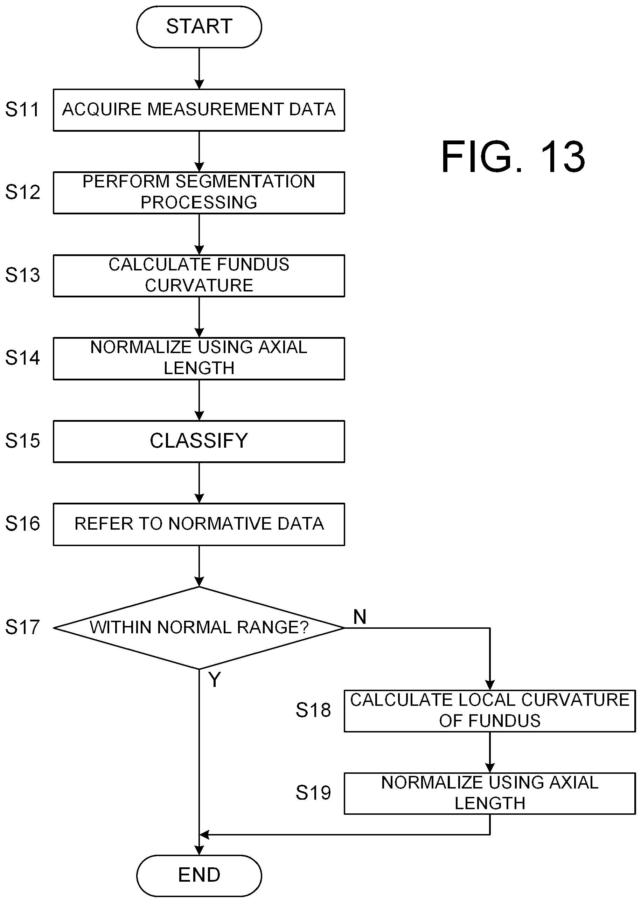

FIG. 13 shows the second operation example of the ophthalmic apparatus 1000. FIG. 13 represents a flowchart of the second operation example of the ophthalmic apparatus 1000. The storage unit 212 stores computer program(s) for realizing the processing shown in FIG. 13. The main controller 211 operates according to the computer program(s), and thereby the main controller 211 performs the processing shown in FIG. 13. In the following, it is assumed that position matching of the optical system relative to the subject's eye E has already been completed. Further, it is assumed that the storage unit 212 stores the normative data indicating the relationship between the fundus curvatures normalized using the axial lengths and the abnormal ranges (normal ranges) of the eye shape.

( S11: Acquire measurement data)

First, the main controller 211 controls the OCT optical system 8 to perform OCT scan on the scan range set on the fundus Ef of the subject's eye E, in the same way as in step S1. With this, the two-dimensional or three-dimensional OCT data is acquired as measurement data.

( S12: Perform segmentation processing )

Next, the main controller 211 controls the segmentation processor 251 to perform segmentation processing on the measurement data acquired in step S11, in the same way as in step S2.

( S13: Calculate fundus curvature )

Next, the main controller 211 controls the fundus shape calculator 253 to calculate the curvature of the RPE layer as the fundus curvature from the shape profile of the RPE layer obtained in step S12, in the same way as in step S3.

( S14: Normalize using axial length )

Next, the main controller 211 controls the first normalizer 254 to normalize the fundus curvature calculated in step S3, using the axial length of the subject's eye E, in the same way as in step S4.

( S15: Classify)

Subsequently, the main controller 211 controls the classifier 256 to classify the eye shape of the subject's eye E into any of the eye shape type 1 to the eye shape type 4 shown in FIG. 7.

For example, the classifier 256 identifies the eye shape type of the subject's eye E by determining whether the fundus curvature normalized using the axial length in step S14 falls within any of the ranges P1 to P4 shown in FIG. 8.

( S16: Refer to normative data)

Next, the main controller 211 controls the determiner 255 to refer to the normative data stored in the storage unit 212, in the same way as in step S6.

( S17: Within normal range? )

Subsequently, the main controller 211 controls the determiner 255 to determine whether or not the eye shape of the subject's eye is within the normal range by comparing the normative data referred in step S16 with the normalized fundus curvature acquired in step S14, in the same way as in step S7.

When it is determined in step S17 that the eye shape of the subject's eye E is within normal range (S17: Y), the ophthalmic apparatus 1000 terminates the operation (END). When it is determined in step S17 that the eye shape of the subject's eye E is not within the normal range (S17: N), the operation of the ophthalmic apparatus 1000 proceeds to step S18.

( S18: Calculate local curvature of fundus )

When it is determined in step S17 that the eye shape of the subject's eye E is not within the normal range (S17: N), the main controller 211 controls the local shape calculator 261 to calculate a plurality of local curvatures of the RPE layer from the shape profile of the RPE layer acquired in step S12.

( S19: Normalize using axial length )

Subsequently, the main controller 211 controls the second normalizer 262 to normalize each of the plurality of local curvatures calculated in step S18, using the axial length of the subject's eye E. The second normalizer 262 normalizes the local curvatures as shown in Equation (1) or Equation (2).

For example, the main controller 211 causes the display unit 270 to display a profile of the local curvatures normalized using the axial length in step S19, as shown in FIG. 10.

This allows the examiner or physician, etc. to identify the eye shape or the change in the eye shape that is difficult to determine by simply referring to a general shape profile. For example, the examiner or physician, etc. can determine whether or not the subject's eye E is high myopia or pathologic myopia from the morphology of the abnormal portion of the eye shape. For example, when the position of the abnormal portion of the eye shape is identified and the portion is judged to be changing slowly, the examiner or physician, etc. can judge that the condition is not serious and can prescribe eyeglasses or contact lenses for the subject. For example, when the position of the abnormal portion of the eye shape is in the macula or the periphery of the macula, the examiner or physician, etc. can determine that there is a possibility of posterior staphyloma, etc. and urge the subject to undergo a visual function test.

This terminates the second operation example of the ophthalmic apparatus 1000 (END).

[ Third operation example ]

A third operation example is an operation example when the normalized shape data of the fundus Ef is used as a single indicator to analyze the eye shape type of the subject's eye E in detail and to identify the eye shape type of the subject's eye E.

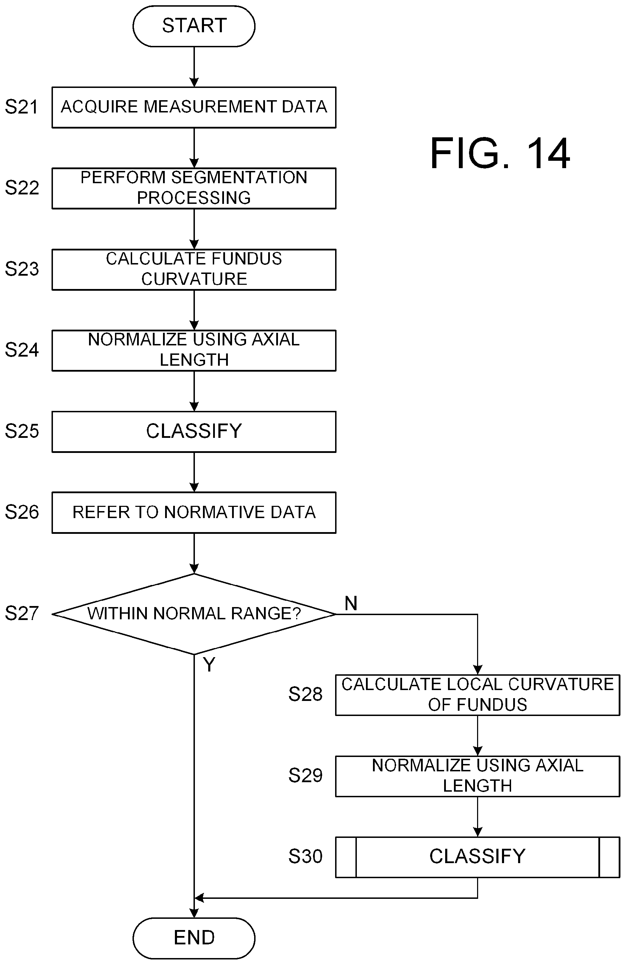

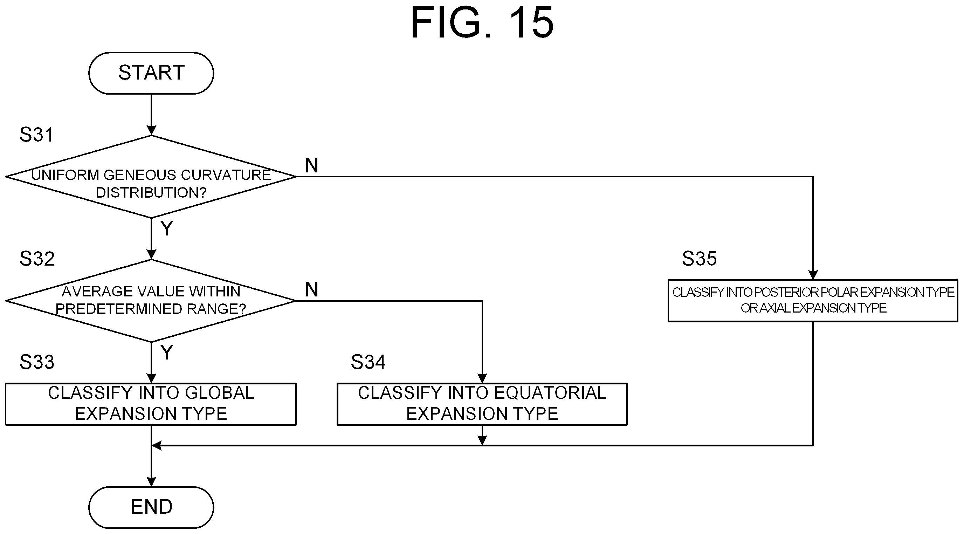

FIG. 14 and FIG. 15 show the third operation example of the ophthalmic apparatus 1000. FIG. 14 represents a flowchart of the third operation example of the ophthalmic apparatus 1000. FIG. 15 shows a flowchart of an example of the operation of step S29 in FIG. 14. The storage unit 212 stores computer program(s) for realizing the processing shown in FIG. 14 and FIG. 15. The main controller 211 operates according to the computer program(s), and thereby the main controller 211 performs the processing shown in FIG. 14 and FIG. 15. In the following, it is assumed that position matching of the optical system relative to the subject's eye E has already been completed. Further, it is assumed that the storage unit 212 stores the normative data indicating the relationship between the fundus curvatures normalized using the axial lengths and the abnormal ranges (normal ranges) of the eye shape.

( S21: Acquire measurement data)

First, the main controller 211 controls the OCT optical system 8 to perform OCT scan on the scan range set on the fundus Ef of the subject's eye E, in the same way as in step S1. With this, the two-dimensional or three-dimensional OCT data is acquired as measurement data.

( S22: Perform segmentation processing)

Next, the main controller 211 controls the segmentation processor 251 to perform segmentation processing on the measurement data acquired in step S21, in the same way as in step S2.

( S23: Calculate fundus curvature )

Next, the main controller 211 controls the fundus shape calculator 253 to calculate the curvature of the RPE layer as the fundus curvature from the shape profile of the RPE layer obtained in step S22, in the same way as in step S3.

( S24: Normalize using axial length )

Next, the main controller 211 controls the first normalizer 254 to normalize the fundus curvature calculated in step S23, using the axial length of the subject's eye E, in the same way as in step S4.

( S25: Classify)

Subsequently, the main controller 211 controls the classifier 256 to classify the eye shape of the subject's eye E into any of the eye shape type 1 to the eye shape type 4 shown in FIG. 7.

For example, the classifier 256 identifies the eye shape type of the subject's eye E by determining whether the fundus curvature normalized using the axial length in step S24 falls within any of the ranges P1 to P4 shown in FIG. 8.

( S26: Refer to normative data)

Next, the main controller 211 controls the determiner 255 to refer to the normative data stored in the storage unit 212, in the same way as in step S6.

( S27: Within normal range? )

Subsequently, the main controller 211 controls the determiner 255 to determine whether or not the eye shape of the subject's eye is within the normal range by comparing the normative data referred in step S26 with the normalized fundus curvature acquired in step S24, in the same way as in step S7.

When it is determined in step S27 that the eye shape of the subject's eye E is within normal range (S27: Y), the ophthalmic apparatus 1000 terminates the operation (END). When it is determined in step S27 that the eye shape of the subject's eye E is not within the normal range (S27: N), the operation of the ophthalmic apparatus 1000 proceeds to step S28.

( S28: Calculate local curvature of fundus )

When it is determined in step S27 that the eye shape of the subject's eye E is not within the normal range (S27: N), the main controller 211 controls the local shape calculator 261 to calculate a plurality of local curvatures of the RPE layer from the shape profile of the RPE layer acquired in step S22, in the same way as in step S18.

( S29: Normalize using axial length )

Subsequently, the main controller 211 controls the second normalizer 262 to normalize each of the plurality of local curvatures calculated in step S28, using the axial length of the subject's eye E, in the same way as in step S19.

( S30: Classify)

Subsequently, the main controller 211 controls the classifier 256 to classify the eye shape of the subject's eye E into any of the eye shape type 1 to the eye shape type 4 shown in FIG. 7, based on the local plurality of local curvatures acquired in step S29. In other words, the eye shape is classified using a single indicator in step S25, and when it is determined not to be normal, the eye shape is reclassified by local analysis. The details of step S30 will be described below.

This terminates the third operation example of the ophthalmic apparatus 1000 (END).

In step S30 in FIG. 14, for example, the following processing is performed.

( S31: Uniform curvature distribution? )

In step S30 in FIG. 14, the classifier 256 determines whether or not the distribution of the normalized local curvatures acquired in step S29 is uniform. For example, the classifier 256 obtains the standard deviation or the variance of the plurality of normalized local curvatures acquired in step S29, and determines whether or not the distribution of the local curvatures is uniform based on the obtained standard deviation or variance.

When it is determined in step S31 that the distribution of the curvatures is uniform (S31: Y), the operation of step S30 proceeds to step S32. When it is determined in step S31 that the distribution of the curvatures is not uniform (S31: N), the operation of step S30 proceeds to step S35.

( S32: Average value within predetermined range? )

When it is determined in step S31 that the distribution of the curvatures is uniform (S31: Y), the classifier 256 obtains the average value of the plurality of normalized local curvatures acquired in step S29 and determines whether or not the obtained average value is within a predetermined range.

When it is determined in step S32 that the average value is within the predetermined range (S32: Y), the operation of step S30 proceeds to step S33. When it is determined in step S32 that the distribution of the curvatures is not within the predetermined range (S32: N), the operation of step S30 proceeds to step S34.

( S33: Classify into Global expansion type)

When it is determined in step S32 that the average value is within the predetermined range (S32: Y), the classifier 256 classifies the eye shape of the subject's eye E into the Global expansion type (eye shape type 1). This terminates the processing of step S30 (END).

( S34: Classify into Equatorial expansion type)

When it is determined in step S32 that the average value is not within the predetermined range (S32: N), the classifier 256 classifies the eye shape of the subject's eye E into the Equatorial expansion type (eye shape type 2). This terminates the processing of step S30 (END).

( S35: Classify into Posterior polar expansion type or Axial expansion type)

When it is determined in step S31 that the curvature distribution is not uniform (S31: N), the classifier 256 classifies the eye shape of the subject's eye E into the Posterior polar expansion type (eye shape type 3) or the Axial expansion type (eye shape type 4). In some embodiments, the classifier 256 further classifies the eye shape of the subject's eye E into the Posterior polar expansion type (eye shape type 3) or the Axial expansion type (eye shape type 4) based on the fundus curvature normalized by the first normalizer 254. This terminates the processing of step S30 (END).

[ Fourth operation example ]

A fourth operation example is an operation example when the acquired measurement data is normalized using the axial length, the normalized shape data of the fundus Ef is calculated from the normalized measurement data, and the calculated shape data is used as a single indicator to identify the eye shape type of the subject's eye E.

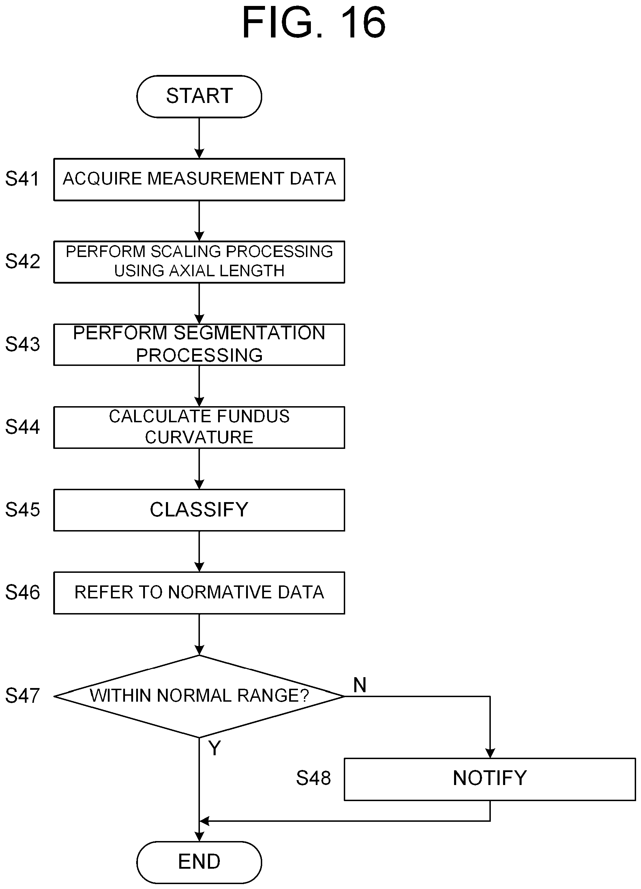

FIG. 16 shows the fourth operation example of the ophthalmic apparatus 1000. FIG. 16 represents a flowchart of the fourth operation example of the ophthalmic apparatus 1000. The storage unit 212 stores computer program(s) for realizing the processing shown in FIG. 16. The main controller 211 operates according to the computer program(s), and thereby the main controller 211 performs the processing shown in FIG. 16. In the following, it is assumed that position matching of the optical system relative to the subject's eye E has already been completed. Further, it is assumed that the storage unit 212 stores the normative data indicating the relationship between the fundus curvatures normalized using the axial lengths and the abnormal ranges (normal ranges) of the eye shape.

( S41: Acquire measurement data)

First, the main controller 211 controls the OCT optical system 8 to perform OCT scan on the scan range set on the fundus Ef of the subject's eye E, in the same way as in step S1. With this, the two-dimensional or three-dimensional OCT data is acquired as measurement data.

( S42: Perform scaling processing using axial length )

Subsequently, the main controller 211 controls the first normalizer 254 to perform scaling processing on the measurement data acquired in step S41 using the axial length. Here, the scaling means extending the unit length of the coordinate axes in the coordinate system defining the measurement data according to the axial length. The scaling is an example of normalization according to the embodiments.

( S43: Perform segmentation processing)

Next, the main controller 211 controls the segmentation processor 251 to perform segmentation processing on the measurement data on which the scaling processing is performed in step S42. Thereby, the shape profile of the RPE layer is acquired.
It should be noted that the order of performing steps S42 and S43 may be interchanged.

( S44: Calculate fundus curvature )

Next, the main controller 211 controls the fundus shape calculator 253 to calculate the curvature of the RPE layer as the fundus curvature from the shape profile of the RPE layer obtained in step S43, in the same way as in step S3.

( S45: Classify)

Subsequently, the main controller 211 controls the classifier 256 to classify the eye shape of the subject's eye E into any of the eye shape type 1 to the eye shape type 4 shown in FIG. 7.
For example, the classifier 256 identifies the eye shape type of the subject's eye E by determining whether the fundus curvature normalized using the axial length in step S44 falls within any of the ranges P1 to P4 shown in FIG. 8.

( S46: Refer to normative data)

Next, the main controller 211 controls the determiner 255 to refer to the normative data stored in the storage unit 212, in the same way as in step S6.

( S47: Within normal range? )

Subsequently, the main controller 211 controls the determiner 255 to determine whether or not the eye shape of the subject's eye is within the normal range by comparing the normative data referred in step S46 with the normalized fundus curvature acquired in step S44, in the same way as in step S7.
When it is determined in step S47 that the eye shape of the subject's eye E is within normal range (S47: Y), the ophthalmic apparatus 1000 terminates the operation (END). When it is determined in step S47 that the eye shape of the subject's eye E is not within the normal range (S47: N), the operation of the ophthalmic apparatus 1000 proceeds to step S48.

( S48: Notify )

When it is determined in step S47 that the eye shape of the subject's eye E is not within the normal range (S47: N), the main controller 211 notifies the information indicating that the eye shape of the subject's eye E is determined to be abnormal, in the same way as in step S8. This terminates the fourth operation example of the ophthalmic apparatus 1000 (END).

[ Fifth operation example ]

A fifth operation example is an operation example when the time-series data of the normalized shape data of the fundus Ef is used as an indicator to identify the eye shape type of the subject's eye E.

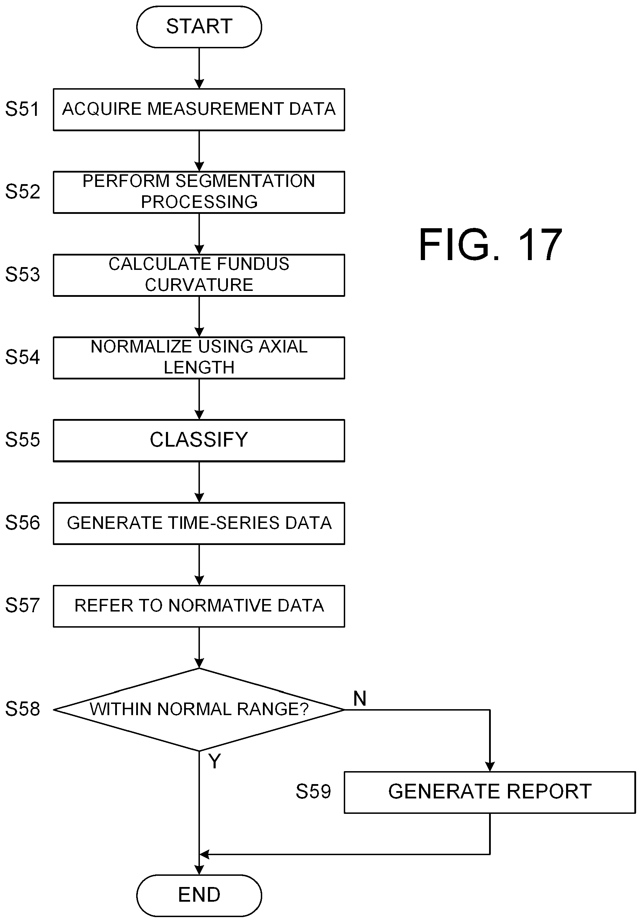

FIG. 17 shows the fifth operation example of the ophthalmic apparatus 1000. FIG. 17 represents a flowchart of the fifth operation example of the ophthalmic apparatus 1000. The storage unit 212 stores computer program(s) for realizing the processing shown in FIG. 17. The main controller 211 operates according to the computer program(s), and thereby the main controller 211 performs the processing shown in FIG. 17. In the following, it is assumed that position matching of the optical system relative to the subject's eye E has already been completed. Further, it is assumed that the storage unit 212 stores the normative data indicating the relationship between the fundus curvatures normalized using the axial lengths and the abnormal ranges (normal ranges) of the eye shape.

( S51: Acquire measurement data)

First, the main controller 211 controls the OCT optical system 8 to perform OCT scan on the scan range set on the fundus Ef of the subject's eye E, in the same way as in step S1. With this, the two-dimensional or three-dimensional OCT data is acquired as measurement data.

( S52: Perform segmentation processing )

Next, the main controller 211 controls the segmentation processor 251 to perform segmentation processing on the measurement data acquired in step S51, in the same way as in step S2.

( S53: Calculate fundus curvature )

Next, the main controller 211 controls the fundus shape calculator 253 to calculate the curvature of the RPE layer as the fundus curvature from the shape profile obtained in step S52, in the same way as in step S3.

( S54: Normalize using axial length )

Next, the main controller 211 controls the first normalizer 254 to normalize the fundus curvature calculated in step S3, using the axial length of the subject's eye E, in the same way as in step S4.

( S55: Classify)

Subsequently, the main controller 211 controls the classifier 256 to classify the eye shape of the subject's eye E into any of the eye shape type 1 to the eye shape type 4 shown in FIG. 7.

For example, the classifier 256 identifies the eye shape type of the subject's eye E by determining whether the fundus curvature normalized using the axial length in step S54 falls within any of the ranges P1 to P4 shown in FIG. 8.

( S56: Generate time-series data)

Next, the main controller 211 controls the data processor 250 to generate the time-series data of the normalized fundus curvatures acquired in step S54.

( S57: Refer to normative data)

Next, the main controller 211 controls the determiner 255 to refer to the normative data stored in the storage unit 212, in the same way as in step S7.

( S58: Within normal range? )

Subsequently, the main controller 211 controls the determiner 255 to determine whether or not the eye shape of the subject's eye is within the normal range by comparing the normative data referred in step S57 with the time-series data of the normalized fundus curvature acquired in step S56, in the same way as in step S7.

When it is determined in step S58 that the eye shape of the subject's eye E is within normal range (S58: Y), the ophthalmic apparatus 1000 terminates the operation (END). When it is determined in step S58 that the eye shape of the subject's eye E is not within the normal range (S58: N), the operation of the ophthalmic apparatus 1000 proceeds to step S59.

( S59: Generate report)

When it is determined in step S58 that the eye shape of the subject's eye E is not within the normal range (S58: N), the main controller 211 controls the report generator 257 to generate the report. The report generator 257 can generate the report showing the classification result(s) in step S55 or the relationship between the normalized fundus curvature and the normal range of the eye shape. The report generator 257 can generate the report (warnings, future forecast, etc.) corresponding to the relative position of the normalized fundus curvature to the normal range of the eye shape. It should be noted that when it is determined in step S58 that the eye shape of the subject's eye E is within the normal range (S58: Y), the main controller 211 may control the report generator 257 to generate the report. Also in this case, the report generator 257 can generate the report (warnings, future forecast, etc.) corresponding to the classification result(s) in step S55, the relationship between the normalized fundus curvature and the normal range of the eye shape, or the relative position of the normalized fundus curvature to the normal range of the eye shape.

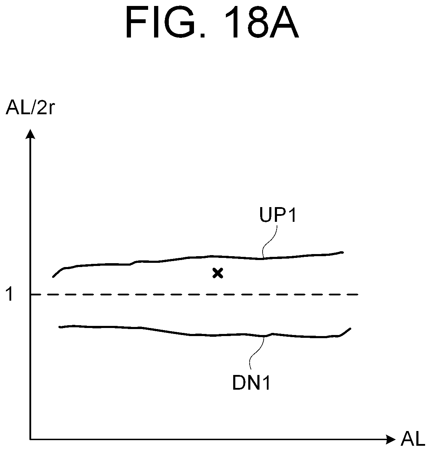

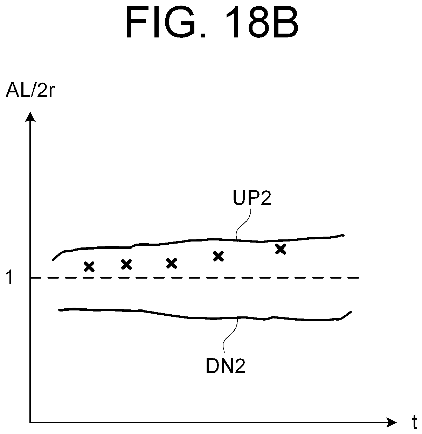

FIG. 18A and FIG. 18B show examples of graphs included in the report generated by the report generator 257. FIG. 18A represents the graph showing the relationship between the normalized fundus curvature and the normal range of the eye shape. In FIG. 18A, the horizontal axis represents the axial length, and the vertical axis represents the normalized fundus curvature ($= AL / 2r$).

The determiner 255 determines whether or not the eye shape of the subject's eye E is normal with reference to the normative data, as described above. Further, when the normalized fundus curvature is between the upper limit UP1 and the lower limit DN1 of the normal range, the determiner 255 can determine that the fundus shape of the subject's eye E is normal. And, when the normalized fundus curvature is above the upper limit UP1 or below the lower limit DN1 of the normal range, the determiner 255 can determine that the fundus shape of the subject's eye E is abnormal.

Furthermore, the determiner 255 can determine the presence or absence of future risk for the change in the eye shape of the subject's eye E by comparing it with the normative data.

FIG. 18B represents the graph showing the relationship between the time-series data of the normalized fundus curvatures and the normal range of the eye shape. In FIG. 18B, the horizontal axis represents the time (date and time of measurement), and the vertical axis represents the normalized fundus curvature ($= AL / 2r$).

When the time-series data of the normalized fundus curvature is between the upper limit UP2 and the lower limit DN2 of the normal range, the determiner 255 can determine that the fundus shape of the subject's eye E is normal. And, when the time-series data of the normalized fundus curvature is above the upper limit UP2 or below the lower limit DN2 of the normal range, the determiner 255 can determine that the fundus shape of the subject's eye E is abnormal.

The determiner 255 can analyze the similarity between the time-series data of the fundus curvature of the subject's eye E and the change(s) in fundus curvature characteristic of high myopia in the shape database prepared in advance to determine the presence or absence of future high myopia risk. Furthermore, the determiner 255 can determine the speed of progression of shape change in the future or the trend of shape change in the future from time-series data of the fundus curvatures.

The report generator 257 can generate the report including the determination result(s) obtained by such determiner 255.

This terminates the first operation example of the ophthalmic apparatus 1000 (END).

[ Actions ]

The ophthalmic information processing apparatus, the ophthalmic apparatus, the ophthalmic information processing method, and the program according to the embodiments will be described.

An ophthalmic information processing apparatus (ophthalmic apparatus 1000, or data processor 250 and communication unit 290) according to the embodiments includes an acquisition unit (optical system shown in FIG. 1 and FIG. 2 (in particular, OCT optical system 8) or communication unit 290) and a normalizer (first normalizer 254, second normalizer 262). The acquisition unit is configured to acquire eye shape data or an intraocular distance of an eye of a subject (subject's eye E). The normalizer is configured to normalize the eye shape data or the intraocular distance based on body data of the subject or a refractive power of the eye of the subj ect.

According to such an aspect, the eye shape or the change in the eye shape can be identified reproducibly and with high precision, without being affected by growth factors of the subject (eye of the subject).

An ophthalmic information processing apparatus (ophthalmic apparatus 1000, or data processor 250 and communication unit 290) according to the embodiments includes a normalizer (first normalizer 254, second normalizer 262) and a calculator (fundus shape calculator 253, local shape calculator 261). The normalizer is configured to normalize measurement data (OCT data) of an eye of a subject (subject's eye E) based on body data of the subject or a refractive power of the eye of the subject. The calculator is configured to calculate normalized eye shape data or a normalized intraocular distance of the eye of the subject based on the measurement data normalized by the normalizer.

According to such an aspect, the eye shape or the change in the eye shape can be identified reproducibly and with high precision, without being affected by growth factors of the subject (eye of the subject).

In some embodiments, the eye shape data includes at least one of a curvature of an anterior segment of the eye of the subject, a radius of curvature of the anterior segment, a curvature of a posterior segment of the eye of the subject, or a radius of curvature of the posterior segment.

According to such an aspect, the curvature or the radius of curvature of the anterior segment or the posterior segment of the eye of the subject, or the change in the curvature or the radius of curvature can be identified reproducibly and with high precision, without being affected by growth factors of the subject (eye of the subject).

In some embodiments, the intraocular distance includes a distance between a macula and an optic disc in the eye of the subject.

According to such an aspect, the distance between the macula and the optic disc in the eye of the subject or the change in the distance between the macula and the optic disc in the eye of the subject can be identified reproducibly and with high precision, without being affected by growth factors of the subject (eye of the subject).

In some embodiments, the body data includes an axial length of the eye of the subject.

According to such an aspect, the eye shape or the change in the eye shape can be reproducibly identified with a simple processing and with high precision, without being affected by growth factors of the subject's eye.

The ophthalmic information processing apparatus according to some embodiments further includes a display controller (controller 210, main controller 211) configured to display two or more of the eye shape data or two or more of the intraocular distances, whose measurement timings are different from each other, in time series on a display means (display unit 270).

According to such an aspect, the time series variation of the eye shape data or the intraocular distance can be easily identified reproducibly and with high precision, without being affected by growth factors of the subject (eye of the subject).

The ophthalmic information processing apparatus according to some embodiments further includes a determiner (255) configured to determine whether or not an eye shape of the eye of the subject is abnormal based on the eye shape data normalized by the normalizer or the eye shape data calculated by the calculator.

According to such an aspect, it is possible to determine reproducibly and with high precision whether or not the eye shape of the eye of the subject is abnormal, without being affected by growth factors of the subject (eye of the subject).

The ophthalmic information processing apparatus according to some embodiments further includes a notifier (controller 210, main controller 211) configured to notify abnormality of the eye shape of the eye of the subject when it is determined by the determiner that the eye shape of the eye of the subject is abnormal.

According to such an aspect, it is possible to notify the abnormality of the eye shape of the eye of the subject reproducibly and with high precision, without being affected by growth factors of the subject (eye of the subject). Thereby, appropriate treatment can be given to the subject at an early stage.

In some embodiments, the normalizer is configured to normalize local eye shape data at an abnormal site or an intraocular distance based on the body data or the refractive power, when it is determined by the determiner that the eye shape of the eye of the subject is abnormal.

According to such an aspect, the abnormal site of the eye shape can be analyzed reproducibly and with high precision, without being affected by growth factors of the subject (eye of the subject).

The ophthalmic information processing apparatus according to some embodiments further includes a classifier (255) configure to classify the eye shape of the eye of the subject into any of a plurality of eye shape types (eye shape type 1 to eye shape type 4) determined in advance, based on the local eye shape data normalized by the normalizer.

According to such an aspect, the predetermined eye shape type can be identified and an appropriate treatment corresponding to the identified eye shape type can be determined, without being affected by growth factors of the subject (eye of the subject).

An ophthalmic apparatus (1000) according to the embodiments includes a measurement system (optical system shown in FIG. 1 and FIG. 2 (in particular, optical system 8) configure to measure an eye shape or an intraocular distance of the eye of the subject and the ophthalmic information processing apparatus in any one of the above.

According to such an aspect, the ophthalmic apparatus that can identify the eye shape or the change in the eye shape reproducibly and with high precision, without being affected by growth factors of the subject (eye of the subject), can be provided.

An ophthalmic information processing method according to the embodiments includes an acquisition step and a normalization step. The acquisition step is performed to acquire eye shape data or an intraocular distance of an eye of a subject (subject's eye E). The normalization step is performed to normalize the eye shape data or the intraocular distance based on body data of the subject or a refractive power of the eye of the subject.

According to such an aspect, the eye shape or the change in the eye shape can be identified reproducibly and with high precision, without being affected by growth factors of the subject (eye of the subject).

An ophthalmic information processing method according to the embodiments includes a normalization step and a calculation step. The normalization step is performed to normalize measurement data (OCT data) of an eye of a subject based on body data of the subject or a refractive power of the eye of the subject (subject's eye E). The calculation step is performed to calculate normalized eye shape data or a normalized intraocular distance of the eye of the subject based on the measurement data normalized in the normalization step.

According to such an aspect, the eye shape or the change in the eye shape can be identified reproducibly and with high precision, without being affected by growth factors of the subject (eye of the subject).

In some embodiments, the eye shape data includes at least one of a curvature of an anterior segment of the eye of the subject, a radius of curvature of the anterior segment, a curvature of a posterior segment of the eye of the subject, or a radius of curvature of the posterior segment.

According to such an aspect, the curvature or the radius of curvature of the anterior segment or the posterior segment of the eye of the subject, or the change in the curvature or the radius of curvature can be identified reproducibly and with high precision, without being affected by growth factors of the subject (eye of the subject).

In some embodiments, the intraocular distance includes a distance between a macula and an optic disc in the eye of the subject.

According to such an aspect, the distance between the macula and the optic disc in the eye of the subject or the change in the distance between the macula and the optic disc in the eye of the subject can be identified reproducibly and with high precision, without being affected by growth factors of the subject (eye of the subject).

In some embodiments, the body data includes an axial length of the eye of the subject.

According to such an aspect, the eye shape or the change in the eye shape can be reproducibly identified with a simple processing and with high precision, without being affected by growth factors of the subject's eye.

The ophthalmic information processing method further includes a display control step of displaying two or more of the eye shape data or two or more of the intraocular distances, whose measurement timings are different from each other, in time series on a display means (display unit 270).

According to such an aspect, the time series variation of the eye shape data or the intraocular distance can be easily identified reproducibly and with high precision, without being affected by growth factors of the subject (eye of the subject).

The ophthalmic information processing method further includes a determination step of determining whether or not an eye shape of the eye of the subject is abnormal based on the eye shape data normalized in the normalization step or the eye shape data calculated in the calculation step.

According to such an aspect, it is possible to determine reproducibly and with high precision whether or not the eye shape of the eye of the subject is abnormal, without being affected by growth factors of the subject (eye of the subject).

The ophthalmic information processing method further includes a notification step of notifying abnormality of the eye shape of the eye of the subject when it is determined in the determination step that the eye shape of the eye of the subject is abnormal.

According to such an aspect, it is possible to notify the abnormality of the eye shape of the eye of the subject reproducibly and with high precision, without being affected by growth factors of the subject (eye of the subject). Thereby, appropriate treatment can be given to the subject at an early stage.

The ophthalmic information processing method further includes a local normalization step of normalizing local eye shape data at an abnormal site or an intraocular distance based on the body data or the refractive power, when it is determined in the determination step that the eye shape of the eye of the subject is abnormal.

According to such an aspect, the abnormal site of the eye shape can be analyzed reproducibly and with high precision, without being affected by growth factors of the subject (eye of the subject).

The ophthalmic information processing method further includes a classification step of classifying the eye shape of the eye of the subject into any of a plurality of eye shape types (eye shape type 1 to eye shape type 4) determined in advance, based on the local eye shape data normalized in the normalization step.

According to such an aspect, the predetermined eye shape type can be identified and an appropriate treatment corresponding to the identified eye shape type can be determined, without being affected by growth factors of the subject (eye of the subject).

A program according to the embodiments causes a computer to execute each step of the ophthalmic information processing method of any one of described above.

According to such an aspect, the eye shape or the change in the eye shape can be identified reproducibly and with high precision, without being affected by growth factors of the subject (eye of the subject).

< Others >

In the embodiments described above, the method of evaluating the fundus shape or the change in the fundus shape in the posterior segment (in particular, fundus Ef) has been described. However, the configuration according to the embodiments is not limited thereto. For example, the eye shape or the change in the eye shape in the anterior segment can be evaluated in the same way as in the embodiments described above. For example, the eye shape or the change in the eye shape in the equatorial part can be evaluated in the same way as in the embodiments described above.

A program for realizing the ophthalmic information processing method according to some embodiments can be stored in any kind of non-transitory computer-readable recording medium. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like.

The computer program may be transmitted and received through a network such as the Internet, LAN, etc.

The above-described embodiments are merely examples for carrying out the present invention. Those who intend to implement the present invention can apply any modification, omission, addition, or the like within the scope of the gist of the present invention.

[EXPLANATION OF SYMBOLS]

| | |
|---|---|
| 8 | OCT optical system |
| 9 | Processor |
| 210 | Controller |
| 211 | Main controller |
| 250 | Data processor |
| 251 | Segmentation processor |
| 252 | Intraocular distance calculator |
| 253 | Fundus shape calculator |
| 254 | First normalizer |
| 255 | Determiner |
| 256 | Classifier |
| 257 | Report generator |
| 260 | Local analyzer |
| 261 | Local shape calculator |
| 262 | Second normalizer |
| 1000 | Ophthalmic apparatus |

**Claims**

1. An ophthalmic information processing apparatus, comprising:

   an acquisition unit configured to acquire eye shape data or an intraocular distance of an eye of a subject; and
   a normalizer configured to normalize the eye shape data or the intraocular distance based on body data of the subject or a refractive power of the eye of the subject.

2. An ophthalmic information processing apparatus, comprising:

   a normalizer configured to normalize measurement data of an eye of a subject based on body data of the subject or a refractive power of the eye of the subject; and
   a calculator configured to calculate normalized eye shape data or a normalized intraocular distance of the eye of the subject based on the measurement data normalized by the normalizer.

3. The ophthalmic information processing apparatus of claim 1 or 2, wherein

the eye shape data includes at least one of a curvature of an anterior segment of the eye of the subject, a radius of curvature of the anterior segment, a curvature of a posterior segment of the eye of the subject, or a radius of curvature of the posterior segment.

4.  The ophthalmic information processing apparatus of any one of claims 1 to 3, wherein
    the intraocular distance includes a distance between a macula and an optic disc in the eye of the subject.

5.  The ophthalmic information processing apparatus of any one of claims 1 to 4, wherein
    the body data includes an axial length of the eye of the subject.

6.  The ophthalmic information processing apparatus of any one of claims 1 to 5, further comprising
    a display controller configured to display two or more of the eye shape data or two or more of the intraocular distances, whose measurement timings are different from each other, in time series on a display means.

7.  The ophthalmic information processing apparatus of any one of claims 1 to 6, further comprising
    a determiner configured to determine whether or not an eye shape of the eye of the subject is abnormal based on the eye shape data normalized by the normalizer or the eye shape data calculated by the calculator.

8.  The ophthalmic information processing apparatus of claim 7, further comprising
    a notifier configured to notify abnormality of the eye shape of the eye of the subject when it is determined by the determiner that the eye shape of the eye of the subject is abnormal.

9.  The ophthalmic information processing apparatus of claim 7 or 8, wherein
    the normalizer is configured to normalize local eye shape data at an abnormal site or an intraocular distance based on the body data or the refractive power, when it is determined by the determiner that the eye shape of the eye of the subject is abnormal.

10. The ophthalmic information processing apparatus of claim 9, further comprising
    a classifier configured to classify the eye shape of the eye of the subject into any of a plurality of eye shape types determined in advance, based on the local eye shape data normalized by the normalizer.

11. An ophthalmic apparatus, comprising:

    a measurement system configured to measure an eye shape or an intraocular distance of the eye of the subject; and

the ophthalmic information processing apparatus of any one of claims 1 to 10.

12. An ophthalmic information processing method, comprising:

    an acquisition step of acquiring eye shape data or an intraocular distance of an eye of a subject; and
    a normalization step of normalizing the eye shape data or the intraocular distance based on body data of the subject or a refractive power of the eye of the subject.

13. An ophthalmic information processing method, comprising:

    a normalization step of normalizing measurement data of an eye of a subject based on body data of the subject or a refractive power of the eye of the subject; and
    a calculation step of calculating normalized eye shape data or a normalized intraocular distance of the eye of the subject based on the measurement data normalized in the normalization step.

14. The ophthalmic information processing method of claim 12 or 13, wherein
    the eye shape data includes at least one of a curvature of an anterior segment of the eye of the subject, a radius of curvature of the anterior segment, a curvature of a posterior segment of the eye of the subject, or a radius of curvature of the posterior segment.

15. The ophthalmic information processing method of any one of claims 12 to 14, wherein
    the intraocular distance includes a distance between a macula and an optic disc in the eye of the subject.

16. The ophthalmic information processing method of any one of claims 12 to 15, wherein
    the body data includes an axial length of the eye of the subject.

17. The ophthalmic information processing method of any one of claims 12 to 16, further comprising
    a display control step of displaying two or more of the eye shape data or two or more of the intraocular distances, whose measurement timings are different from each other, in time series on a display means.

18. The ophthalmic information processing method of any one of claims 12 to 17, further comprising
    a determination step of determining whether or not an eye shape of the eye of the subject is abnormal based on the eye shape data normalized in the normalization step or the eye shape data calculated in the calculation step.

**19.** The ophthalmic information processing method of claim 18, further comprising
a notification step of notifying abnormality of the eye shape of the eye of the subject when it is determined in the determination step that the eye shape of the eye of the subject is abnormal.

**20.** The ophthalmic information processing method of claim 18 or 19, further comprising
a local normalization step of normalizing local eye shape data at an abnormal site or an intraocular distance based on the body data or the refractive power, when it is determined in the determination step that the eye shape of the eye of the subject is abnormal.

**21.** The ophthalmic information processing method of claim 20, further comprising
a classification step of classifying the eye shape of the eye of the subject into any of a plurality of eye shape types determined in advance, based on the local eye shape data normalized in the normalization step.

**22.** A program of causing a computer to execute each step of the ophthalmic information processing method of any one of claims 12 to 21.

FIG. 1

FIG. 2

# FIG. 3

EP 4 289 338 A1

# FIG. 4

OCT OPTICAL SYSTEM — 8

- OPTICAL SCANNER — 88
- OPTICAL PATH LENGTH CHANGING UNIT — 89
- 100
  - LIGHT SOURCE UNIT — 101
  - ATTENUATOR — 105
  - POLARIZATION CONTROLLER — 106
  - ZOOM OPTICAL SYSTEM — 114
  - CCD — 115

# FIG. 5

250

```
┌ ─ ─ ─ ─ ─ ─ ─ ─ ─ ─ ─ ─ ─ ─ ─ ─ ─ ┐
│          DATA PROCESSOR           │
│  ┌─────────────────────────────┐  │
│  │   SEGMENTATION PROCESSOR     │  │── 251
│  └─────────────────────────────┘  │
│  ┌─────────────────────────────┐  │
│  │ INTRAOCULAR DISTANCE CALCULATOR│ │── 252
│  └─────────────────────────────┘  │
│  ┌─────────────────────────────┐  │
│  │   FUNDUS SHAPE CALCULATOR    │  │── 253
│  └─────────────────────────────┘  │
│  ┌─────────────────────────────┐  │
│  │      FIRST NORMALIZER        │  │── 254
│  └─────────────────────────────┘  │
│  ┌─────────────────────────────┐  │
│  │      LOCAL ANALYZER          │  │── 260
│  └─────────────────────────────┘  │
│  ┌─────────────────────────────┐  │
│  │       DETERMINER             │  │── 255
│  └─────────────────────────────┘  │
│  ┌─────────────────────────────┐  │
│  │       CLASSIFIER             │  │── 256
│  └─────────────────────────────┘  │
│  ┌─────────────────────────────┐  │
│  │     REPORT GENERATOR         │  │── 257
│  └─────────────────────────────┘  │
└ ─ ─ ─ ─ ─ ─ ─ ─ ─ ─ ─ ─ ─ ─ ─ ─ ─ ┘
```

# FIG. 6

260

```
┌ ─ ─ ─ ─ ─ ─ ─ ─ ─ ─ ─ ─ ─ ─ ─ ─ ─ ┐
│         LOCAL ANALYZER             │
│  ┌──────────────────────────────┐  │
│  │   LOCAL SHAPE CALCULATOR     │  │── 261
│  └──────────────────────────────┘  │
│  ┌──────────────────────────────┐  │
│  │     SECOND NORMALIZER        │  │── 262
│  └──────────────────────────────┘  │
└ ─ ─ ─ ─ ─ ─ ─ ─ ─ ─ ─ ─ ─ ─ ─ ─ ─ ┘
```

# FIG. 7

| EYE SHAPE TYPE 1<br>(GLOBAL EXPANSION TYPE) | EYE SHAPE TYPE 2<br>(EQUATORIAL EXPANSION TYPE) | EYE SHAPE TYPE 3<br>(POSTERIOR POLAR EXPANSION TYPE) | EYE SHAPE TYPE 4<br>(AXIAL EXPANSION TYPE) |
|---|---|---|---|
| | | | |

EP 4 289 338 A1

# FIG. 8

# FIG. 9

# FIG. 10

# FIG. 11A

Retinal shape profile

Radius of curvature distribution

# FIG. 11B

Retinal shape profile

Radius of curvature distribution

# FIG. 11C

Retinal shape profile

Radius of curvature distribution

# FIG. 12

START

S1 | ACQUIRE MEASUREMENT DATA

S2 | PERFORM SEGMENTATION PROCESSING

S3 | CALCULATE FUNDUS CURVATURE

S4 | NORMALIZE USING AXIAL LENGTH

S5 | CLASSIFY

S6 | REFER TO NORMATIVE DATA

S7 | WITHIN NORMAL RANGE? — N

Y

S8 | NOTIFY

END

FIG. 13

```
              ┌─────────────┐
              │    START    │
              └─────────────┘
                     │
                     ▼
S11    ┌──────────────────────────────┐
       │   ACQUIRE MEASUREMENT DATA    │
       └──────────────────────────────┘
                     │
                     ▼
S12    ┌──────────────────────────────┐
       │   PERFORM SEGMENTATION        │
       │        PROCESSING             │
       └──────────────────────────────┘
                     │
                     ▼
S13    ┌──────────────────────────────┐
       │      CALCULATE FUNDUS         │
       │        CURVATURE              │
       └──────────────────────────────┘
                     │
                     ▼
S14    ┌──────────────────────────────┐
       │    NORMALIZE USING AXIAL      │
       │          LENGTH               │
       └──────────────────────────────┘
                     │
                     ▼
S15    ┌──────────────────────────────┐
       │          CLASSIFY             │
       └──────────────────────────────┘
                     │
                     ▼
S16    ┌──────────────────────────────┐
       │    REFER TO NORMATIVE DATA    │
       └──────────────────────────────┘
                     │
                     ▼
S17         ◇ WITHIN NORMAL RANGE? ◇ ──── N
                     │                      │
                     Y                      ▼
                     │        S18  ┌──────────────────────────────┐
                     │             │  CALCULATE LOCAL CURVATURE    │
                     │             │        OF FUNDUS              │
                     │             └──────────────────────────────┘
                     │                      │
                     │                      ▼
                     │        S19  ┌──────────────────────────────┐
                     │             │   NORMALIZE USING AXIAL       │
                     │             │          LENGTH               │
                     │             └──────────────────────────────┘
                     │                      │
                     ▼◄─────────────────────┘
              ┌─────────────┐
              │     END     │
              └─────────────┘
```

START

S21 | ACQUIRE MEASUREMENT DATA

S22 | PERFORM SEGMENTATION PROCESSING

S23 | CALCULATE FUNDUS CURVATURE

S24 | NORMALIZE USING AXIAL LENGTH

S25 | CLASSIFY

S26 | REFER TO NORMATIVE DATA

S27 WITHIN NORMAL RANGE? — N

Y

S28 | CALCULATE LOCAL CURVATURE OF FUNDUS

S29 | NORMALIZE USING AXIAL LENGTH

S30 | CLASSIFY

END

# FIG. 14

# FIG. 15

START

S31 UNIFORM GENEOUS CURVATURE DISTRIBUTION? — N →

S35 CLASSIFY INTO POSTERIOR POLAR EXPANSION TYPE OR AXIAL EXPANSION TYPE

Y

S32 AVERAGE VALUE WITHIN PREDETERMINED RANGE? — N →

S34 CLASSIFY INTO EQUATORIAL EXPANSION TYPE

Y

S33 CLASSIFY INTO GLOBAL EXPANSION TYPE

END

EP 4 289 338 A1

# FIG. 16

START

S41 ACQUIRE MEASUREMENT DATA

S42 PERFORM SCALING PROCESSING USING AXIAL LENGTH

S43 PERFORM SEGMENTATION PROCESSING

S44 CALCULATE FUNDUS CURVATURE

S45 CLASSIFY

S46 REFER TO NORMATIVE DATA

S47 WITHIN NORMAL RANGE? — N

Y

S48 NOTIFY

END

START

S51 | ACQUIRE MEASUREMENT DATA

S52 | PERFORM SEGMENTATION PROCESSING

S53 | CALCULATE FUNDUS CURVATURE

S54 | NORMALIZE USING AXIAL LENGTH

S55 | CLASSIFY

S56 | GENERATE TIME-SERIES DATA

S57 | REFER TO NORMATIVE DATA

S58 | WITHIN NORMAL RANGE? — N

S59 | GENERATE REPORT

Y

END

FIG. 17

# FIG. 18A

# FIG. 18B

## INTERNATIONAL SEARCH REPORT

| International application No. |
| --- |
| PCT/JP2021/004244 |

**A. CLASSIFICATION OF SUBJECT MATTER**
A61B 3/10(2006.01)i
FI: A61B3/10
According to International Patent Classification (IPC) or to both national classification and IPC

**B. FIELDS SEARCHED**

Minimum documentation searched (classification system followed by classification symbols)
A61B3/00-3/12, 3/13-3/16

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2016-158906 A (NIDEK CO., LTD.) 05 September 2016 (2016-09-05) paragraphs [0056]-[0061] | 1-2, 11-13, 22 |
| Y | | 3-11, 14-22 |
| Y | WO 2015/087436 A1 (UNIVERSAL VIEW CO., LTD.) 18 June 2015 (2015-06-18) paragraphs [0170]-[0174] | 3-11, 14-22 |
| Y | JP 2018-20192 A (CANON INC.) 08 February 2018 (2018-02-08) paragraphs [0148], [0159]-[0166] | 3-11, 14-22 |
| Y | JP 2018-523171 A (EYEBRIGHT MEDICAL TECHNOLOGY (BEIJING) CO., LTD.) 16 August 2018 (2018-08-16) paragraphs [0033]-[0035] | 4-11, 15-22 |
| A | JP 2018-79208 A (CANON INC.) 24 May 2018 (2018-05-24) paragraphs [0080]-[0093], [0102] | 1-22 |
| A | JP 2015-89477 A (DAINIPPON PRINTING CO., LTD.) 11 May 2015 (2015-05-11) paragraphs [0095]-[0107] | 1-22 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 04 March 2021 (04.03.2021) | 13 April 2021 (13.04.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

| | | International application No. |
|---|---|---|
| | | PCT/JP2021/004244 |

| Patent Documents referred in the Report | Publication Date | Patent Family | Publication Date |
|---|---|---|---|
| JP 2016-158906 A | 05 Sep. 2016 | (Family: none) | |
| WO 2015/087436 A1 | 18 Jun. 2015 | (Family: none) | |
| JP 2018-20192 A | 08 Feb. 2018 | (Family: none) | |
| JP 2018-523171 A | 16 Aug. 2018 | US 2018/0210229 A1 paragraph [0035] WO 2017/016440 A1 EP 3349055 A1 CN 106707542 A CN 106291976 A CN 106291977 A CN 106353892 A KR 10-2018-0034515 A | |
| JP 2018-79208 A | 24 May 2018 | (Family: none) | |
| JP 2015-89477 A | 11 May 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VERKICHARLA PK et al.** Eye shape and retinal shape, and their relation to peripheral refraction. *Ophthalmic Physiol Opt,* 2012, vol. 32, 184-199

- **MUKA MORIYAMA et al.** Quantitative Analyses of High-Resolution 3D MR Images of Highly Myopic Eyes to Determine Their Shapes. *Investigative Ophthalmology & Visual Science,* July 2012, vol. 53 (8), 4510-4518